# EUROPEAN PATENT SPECIFICATION

(11) **EP 3 814 325 B1**
(45) Date of publication and mention of the grant of the patent: **30.11.2022**
(21) Application number: 19786404.4
(22) Date of filing: 04.09.2019
(51) Int. Cl.: C07D 219/08, C07D 221/12

(54) **A RADIOLABELLED COMPOUND OF QUATERNARY AMMONIUM SALT OF A POLYCYCLIC AROMATIC AMINE, THE USE OF THE RADIOLABELLED COMPOUND IN A DIAGNOSTIC METHOD OF POSITRON EMISSION TOMOGRAPHY, AND A PHARMACEUTICAL COMPOSITION CONTAINING THE RADIOLABELLED COMPOUND OF QUATERNARY AMMONIUM SALT OF A POLYCYCLIC AROMATIC AMINE**
RADIOAKTIV MARKIERTE VERBINDUNG EINES QUATERNÄREN AMMONIUMSALZES EINES POLYCYCLISCHEN AROMATISCHEN AMINS, VERWENDUNG DER RADIOAKTIV MARKIERTEN VERBINDUNG IN EINEM DIAGNOSTISCHEN VERFAHREN DER POSITRONENEMISSIONSTOMOGRAPHIE UND PHARMAZEUTISCHE ZUSAMMENSETZUNG MIT DER RADIOAKTIV MARKIERTEN VERBINDUNG EINES QUATERNÄREN AMMONIUMSALZES EINES POLYCYCLISCHEN AROMATISCHEN AMINS
COMPOSÉ RADIOMARQUÉ DE SEL D'AMMONIUM QUATERNAIRE D'UNE AMINE AROMATIQUE POLYCYCLIQUE, UTILISATION DU COMPOSÉ RADIOMARQUÉ DANS UN PROCÉDÉ DE DIAGNOSTIC DE TOMOGRAPHIE PAR ÉMISSION DE POSITRONS, ET COMPOSITION PHARMACEUTIQUE CONTENANT LE COMPOSÉ RADIOMARQUÉ DE SEL D'AMMONIUM QUATERNAIRE D'UNE AMINE AROMATIQUE POLYCYCLIQUE

(30) Priority: 05.09.2018 PL 42691618
(43) Date of publication of application: 05.05.2021
(73) Proprietor: Synektik S.A., 00-728 Warszawa (PL)
(72) Inventor: TOWPIK, Joanna, 02-654 Warszawa (PL); KRAJEWSKI, Seweryn, Andrzej, 01-312 Warszawa (PL); STECZEK, Lukasz, Marek, 01-354 Warszawa (PL); WLOSTOWSKA, Joanna, 01-491 Warszawa (PL)
(74) Representative: Pankowski, Jacek
(86) International application number: PCT/PL2019/000073
(87) International publication number: WO 2020/050729

(56) References cited:
- WO-A1-2018/207193
- STUDENOV A R ET AL: "Synthesis and properties of ^1^8F-labeled potential myocardial blood flow tracers", NUCLEAR MEDICINE AND BIOLOGY, ELSEVIER, NY, US, vol. 28, no. 6, 1 August 2001 (2001-08-01) , pages 683-693, XP004300996, ISSN: 0969-8051, DOI: 10.1016/S0969-8051(01)00233-5
- CATHERINE HOU ET AL: "Development of a Positron Emission Tomography Radiotracer for Imaging Elevated Levels of Superoxide in Neuroinflammation", ACS CHEMICAL NEUROSCIENCE, vol. 9, no. 3, 3 November 2017 (2017-11-03), pages 578-586, XP55636273, US ISSN: 1948-7193, DOI: 10.1021/acschemneuro.7b00385

## Description

The invention relates to a radioisotope-labelled compound of quaternary ammonium salt of a polycyclic aromatic amine, the use of said compound in a diagnostic method of positron emission tomography, and a pharmaceutical composition containing the radioisotope-labelled compound of a polycyclic quaternary aromatic amine.

Nuclear cardiology based on non-invasive imaging studies using radiopharmaceuticals (radiolabelled molecules) makes it possible to assess the function of the cardiovascular system in a safe, fast and relatively non-expensive fashion. Nuclear cardiology procedures are included in various guidelines for the diagnosis and therapy of coronary artery disease. Hybrid PET-CT (positron emission tomography combined with computed tomography) and SPECT-CT (single photon emission computed tomography combined with computed tomography) methods are two well-established, non-invasive imaging techniques used for cardiological diagnostics.

SPECT-CT is an important, non-invasive, widespread method for imaging myocardial perfusion that provides information on all of the myocardial viability, perfusion and function. To assess perfusion, compounds labelled with technetium-99m (sestamibi and tetrophosmin) and thallium-201 (thallium chloride-201) are used. They are comparable in terms of efficiency in detecting coronary artery disease.

PET-CT is used to evaluate the metabolism of glucose, oxygen, perfusion and receptor function. The PET-CT study is useful in diagnosing myocardial viability, and, in addition, it allows for measuring the myocardial physiological activity. The PET-CT study uses radiopharmaceuticals labelled with positron-emitting radionuclides, such as unstable isotopes of elements essential in the structure of living organisms: oxygen(¹⁵O), nitrogen(¹³N) and carbon(¹¹C). The usability of compounds labelled with said radionuclides is limited to short *T*_{1/2} half-life times of 2 min, 10 min and 20 min for ¹⁵O, ¹³N and ¹¹C, respectively. Another positron-emitting radionuclide is the ¹⁸F fluorine isotope; therefore, ¹⁸F-labelled compounds were also used in PET-CT studies. ¹⁸F-fluorodeoxyglucose (¹⁸F-FDG), which, upon bodily administration, is involved in the metabolic pathway of glucose, is the most common compound used in clinical practice. The ¹⁸F-FDG study allows for monitoring myocardial metabolic changes in patients with chronic heart failure, so that it provides an excellent prognostic tool in chronic heart failure. Since the half-life *T*_{1/2} of the ¹⁸F radioisotope is longer than that of ¹⁵O, ¹³N or ¹¹C (*T*_{1/2} for ¹⁸F is 109.8 min), the ¹⁸F-labelled radiopharmaceutical may be manufactured in locations other than the study performed.

International publication WO 2011/084585 discloses an invention of a radiolabelled dihydroethidine derivative having a triphenylphosphonioalkylene group attached to the nitrogen atom of the heterocyclic ring and a phenyl group attached to the sp³ carbon atom of said heterocyclic ring. The phenyl group is substituted by other substituents in which a fluorine atom may be present. According to one of the aspects of the solution, the radioisotope is an isotope emitting positron radiation, especially ¹¹C or ¹⁸F. The compound according to the invention is designed for visualising the distribution of free oxygen radicals, especially peroxide anion radicals in an animal body, since dihydroethidine derivatives are oxidised by peroxides, and the use of positron emission tomography imaging provides detailed data on the distribution of peroxide anion radicals *in vivo.*

Methods are known for manufacturing selected quaternary aromatic amines containing atoms of ¹⁹F fluorine (i.e. non-radioactive fluorine isotope) in aliphatic substituent of the nitrogen atom of the amine. Patent US 4,062,849 discloses the manufacturing of N-heptadecafluorodecyl acridinium iodide using N-heptadecafluorodecyl iodide (C₈F₁₇C₂H₄I) and a corresponding aromatic amine. Moreover, a scientific article entitled "An unusual substitution reaction directed by an intramolecular re-arrangement" (A.D.C. Parenty, L.V. Smith, L. Cronin), Tetrahedron, 61 (2005), pp. 8410-8418, discloses the manufacturing of 2-fluoroethyl phenanthridinium bromide from 2-fluoroethyl tosylate and phenanthridine with the subsequent exchange of counterion for bromide anion using Dowex 1X-850 resin pre-treated with saturated sodium bromide solution.

The object of the invention is to provide a radioisotope-labelled compound of quaternary ammonium salt of a polycyclic aromatic amine, the use of the radioisotope-labelled compound of quaternary ammonium salt of a polycyclic aromatic amine in a diagnostic method of positron emission tomography, and to provide a pharmaceutical composition containing said radioisotope-labelled compound of quaternary ammonium salt of a polycyclic aromatic amine.

The invention relates to a radioisotope-labelled compound of quaternary ammonium salt of a polycyclic aromatic amine with formula I, 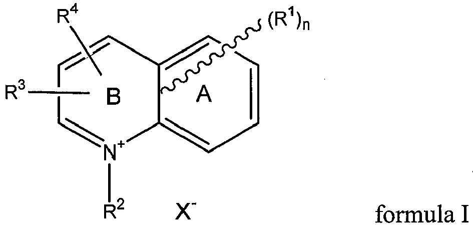 in which formula I
the wavy line indicates a single bond between the non-nodal carbon atom of a polycyclic aromatic system and the R¹ substituent,
R¹ is a substituent independently selected from hydrogen, halogens, C₁₋₄alkoxy, a nitro group, an amino group optionally having 1 or 2 hydrogen atoms replaced with C₁₋₆alkyl or having 2 hydrogen atoms replaced with C₂₋₅alkylene to form a heterocyclic ring, a chain C₁₋₆carbon group optionally having a halogen, carboxy, formyl or C₁₋₄alkanesulfonic substituent, and a phenyl group optionally having 1-5 substituents independently selected from halogens, a chain C₁₋₆carbon substituent, a halogenated chain C₁₋₆carbon substituent, a hydroxy optionally protected, C₁₋₄alkoxy, an amino group optionally having 1-2 hydrogen atoms replaced with C₁₋₆alkyl,
R² is a chain aliphatic substituent having the -CH₂- fragment as the terminal member of the chain, to which chain a phenyl group is optionally attached optionally having 1-3 substituents selected from halogens and C₁₋₆alkyl, and, if the chain contains at least 2 carbon atoms, in which chain there is optionally a bivalent link between the chain carbon atoms, said link selected from the oxygen atom -O-, sulfur atom -S- and C₃₋₆cycloalkylene, wherein the R² substituent contains a total of 1-16 carbon atoms, and the hydrogen atom at one of the carbon atoms is replaced with an atom of ¹⁸F fluorine radioisotope,
R³ and R⁴ are combined to form a bivalent butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
or a hydrate or solvate thereof.

Preferably, the invention relates to a compound with formula I, wherein the wavy line indicates a single bond between the non-nodal carbon atom of a polycyclic aromatic system and the R¹ substituent,
R¹ is a substituent independently selected from hydrogen, halogens, C₁₋₄alkoxy, a nitro group, an amino group optionally having 1 or 2 hydrogen atoms replaced with C₁₋₄alkyl, a chain C₁₋₆carbon group optionally having a halogen or C₁₋₄alkanesulfonic substituent, and a phenyl group optionally having 1-3 substituents independently selected from halogens, a chain C₁₋₄carbon substituent, a halogenated chain C₁₋₄carbon substituent, C₁₋₄alkoxy, an amino group optionally having 1-2 atoms of hydrogen replaced with C₁₋₄alkyl,
R² is a chain aliphatic substituent having the -CH₂- fragment as the terminal member of the chain, to which chain a phenyl group is optionally attached optionally having 1-3 substituents selected from halogens and C₁₋₆alkyl, and, if the chain contains at least 2 carbon atoms, in which chain there is optionally a bivalent link between the carbon atoms, said link selected from the oxygen atom -O- and sulfur atom -S-, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
or a hydrate or solvate thereof.

The invention relates in particular to a compound with formula I, wherein the wavy line indicates a single bond between the non-nodal carbon atom of a polycyclic aromatic system and the R¹ substituent,
R¹ is a substituent independently selected from hydrogen, halogens, C₁₋₄alkoxy, an amino group optionally having 1-2 hydrogen atoms replaced with C₁₋₄alkyl, a chain C₁₋₄carbon group optionally having a halogen substituent, and a phenyl group optionally having 1-3 substituents independently selected from halogens, a chain C₁₋₄carbon substituent, a halogenated chain C₁₋₄carbon substituent, C₁₋₄alkoxy,
R² is a chain aliphatic substituent having the -CH₂- fragment as the terminal member of the chain, to which chain a phenyl group is optionally attached optionally having 1-3 substituents selected from halogens and C₁₋₆alkyl, and, if the chain contains at least 2 carbon atoms, in which chain there is optionally a bivalent link between the carbon atoms, said link being the oxygen atom -O-, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
or a hydrate or solvate.

The invention relates especially to a compound with formula I, wherein the wavy line indicates a single bond between the non-nodal carbon atom of a polycyclic aromatic system and the R¹ substituent,

R¹ is a substituent independently selected from hydrogen, halogens, C₁₋₄alkoxy, an amino group optionally having 1-2 hydrogen atoms replaced with C₁₋₂alkyl, a chain C₁₋₄carbon group optionally having a halogen substituent, and a phenyl group optionally having 1-3 substituents independently selected from halogens, a chain C₁₋₄carbon substituent and a halogenated chain C₁₋₄carbon substituent,
R² is a chain aliphatic substituent having the -CH₂- fragment as the terminal member of the chain, to which chain a phenyl group is optionally attached, optionally having 1-3 substituents selected from halogens and C₁₋₄alkyl, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
or a hydrate or solvate thereof.

Particularly preferably, the invention relates to a compound with formula I, wherein the wavy line indicates a single bond between the non-nodal carbon atom of a polycyclic aromatic system and the R¹ substituent,
R¹ is a substituent independently selected from hydrogen, halogens, an amino group optionally having 1-2 hydrogen atoms replaced with C₁₋₂alkyl, a chain C₁₋₄carbon group, and a phenyl group optionally having 1-3 substituents independently selected from a chain C₁₋₄carbon substituent and a halogenated chain C₁₋₄carbon substituent,
R² is a chain aliphatic substituent having the -CH₂- fragment as the terminal member of the chain, to which chain a phenyl group is optionally attached optionally having a halogen substituent, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
or a hydrate or solvate thereof.

The invention relates to a radioisotope-labelled compound as defined above for use in a diagnostic method of positron emission tomography. Preferably, said diagnostic method is used for the examination of the cardiovascular system in a mammal. Said diagnostic method includes a myocardial perfusion imaging for quantifying regional blood flow through the myocardium and/or a cardiac perfusion test to quantify the coronary reserve in coronary artery disease.

The invention also relates to a pharmaceutical composition containing a radioisotope-labelled compound specified above and a pharmaceutically acceptable carrier or diluent. Preferably, said composition is in the form of a sterile solution.

The radioisotope-labelled compound is used for the manufacture of an agent for use in a diagnostic method of positron emission tomography. Preferably, the diagnostic method is used for the examination of the cardiovascular system in a mammal. The diagnostic method is used, in particular, for testing cardiac perfusion for quantifying regional blood flow through the myocardium and/or for quantifying the coronary reserve in coronary artery disease.

The invention provides a ¹⁸F-radiolabelled compound for use as a cardiotracer to evaluate myocardial perfusion and diagnose coronary artery disease during a PET scan. Use of a cardiotracer containing the ¹⁸F radionuclide, for which the range of positrons of the order of 0.6 mm, allows for obtaining a higher spatial resolution of images acquired during the scan and a higher counting sensitivity compared to other PET tracers, such as ⁸²Rb, where the range of positrons is 5.9 mm or ¹³NH₃ (1.6 mm), since the spatial resolution increases as the kinetic energy of positrons decreases. The use of PET technology and of the cardiotracer according to the invention allows for quantifying myocardial blood flow in absolute values including the estimation of the flow through each coronary artery, without the need for invasive catheterisation thereof. Where cardiac perfusion in a PET test is assessed using tracers containing short half-life radionuclides, cardiac perfusion imaging can only be performed in a PET lab with direct access to a cyclotron or generator, since the tracer activity would usually undergo total decay during the time needed for transport to a remote PET lab. Delivery of a cardiotracer in the form of the compound according to the invention labelled with the ¹⁸F radionuclide, with a half-life of 109.8 min, allows for producing a cardiotracer outside the PET laboratory and, if necessary, for delivering the pharmaceutical form prepared to the place of testing.

Effective and readily accessible cardiological diagnostics using PET is paramount, because, in addition to contributing to understanding the pathophysiology of heart failure, it provides a tool to assess the outcome of pharmacological and invasive treatment. Apart from the data on organ function (perfusion, metabolism and left ventricular function), the PET-CT method provides quantitative data on the significance of anatomical stenoses in coronary arteries. Both tests, when performed simultaneously, provide a comprehensive diagnostic and prognostic method for the evaluation of patients with chronic heart failure of ischemic etiology.

The invention is described in detail with reference to the drawing, in which fig. 1 shows representative PET images of radioactivity distribution obtained in the study on rats following the administration of the pharmaceutical form of 5-(2-[¹⁸F]fluoroethyl)phenanthridinium salt, fig. 2 shows representative PET images of radioactivity distribution obtained in the study on rats following the administration of the pharmaceutical form of 6-phenyl-5-(2-[¹⁸F]fluoroethyl)-phenanthridinium salt, and fig. 3 shows representative PET images obtained in the study on rats following the administration of the pharmaceutical form of 3,6-bis(dimethylamino)-10-(2-[¹⁸F]fluoroethyl)acridinium salt.

The invention relates to a radioisotope-labelled compound of quaternary ammonium salt of a polycyclic aromatic amine with formula I, 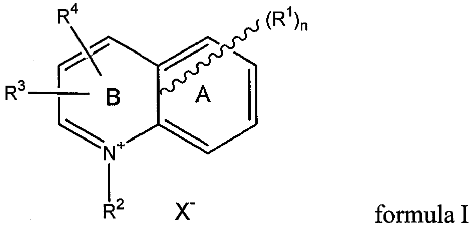 in which formula I
the wavy line indicates a single bond between the non-nodal carbon atom of a polycyclic aromatic system and the R¹substituent,
R¹ is a substituent independently selected from hydrogen, halogens, a hydroxy optionally protected, C₁₋₄alkoxy, a nitro group, an amino group optionally having 1 or 2 hydrogen atoms replaced with C₁₋₆alkyl or having 2 hydrogen atoms replaced with C₂₋₅alkylene to form a heterocyclic ring, a chain C₁₋₆carbon group optionally having a halogen, carboxy, formyl or C₁₋₄alkanesulfonic substituent, and a phenyl group optionally having 1-5 substituents independently selected from halogens, a chain C₁₋₆carbon, a halogenated chain C₁₋₆carbon substituent, a hydroxy optionally protected, C₁₋₄alkoxy, an amino group optionally having 1-2 hydrogen atoms replaced with C₁₋₆alkyl,
R² is a chain aliphatic substituent having the -CH₂- fragment as the terminal member of the chain, to which chain a phenyl group is optionally attached optionally having 1-3 substituents selected from halogens and C₁₋₆alkyl, and, if the chain contains at least 2 carbon atoms, in which chain there is optionally a bivalent link between the chain carbon atoms, said link selected from the oxygen atom -O-, sulfur atom -S- and C₃₋₆cycloalkylene, wherein the R² substituent contains a total of 1-16 carbon atoms, and the hydrogen atom at one of the carbon atoms is replaced with an atom of ¹⁸F fluorine radioisotope,
R³ and R⁴ are combined to form a bivalent butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
and the hydrate or solvate thereof.

The invention also relates to a pharmaceutical composition containing the radioisotope-labelled compound of quaternary ammonium salt of a polycyclic aromatic amine according to the invention and a pharmaceutically acceptable carrier or diluent, in particular to a pharmaceutical composition in the form of a sterile solution. The sterile composition is provided in the final container (vial or syringe) placed in an external shielding.

Compounds according to the invention are manufactured by adapting general synthetic methods available in the field of organic chemistry. The method illustrated in scheme I uses a substituted compound with formula II, wherein R¹, R³ and R⁴ have the meanings indicated above for formula I, and wherein said compound with formula II is a derivative of acridine or phenanthridine, and it uses a compound GO^{A}-CH₂-R⁵(GO^{B}) with formula III, wherein the GO^{A} and GO^{B} symbols are leaving groups substitutable with a nucleophilic reagent in a nucleophilic substitution reaction. The R⁵-CH₂ fragment corresponds in terms of structure to the chain R² substituent defined above. The GO^{A} and GO^{B} leaving groups are structurally identical or different and are independently selected from alkylsulfonate leaving groups, fluoroalkylsulfonate leaving groups, arylsulfonate leaving groups, haloarylsulfonate leaving groups and halogen leaving groups with the exclusion of fluoride group. Preferably, leaving groups are methanesulfonate, ethanesulfonate, trifluoromethanesulfonate (triflate), pentafluoroethanesulfonate, toluenesulfonate (tosyl), 4-bromophenylsulfonate (brosyl), iodide and bromide groups.

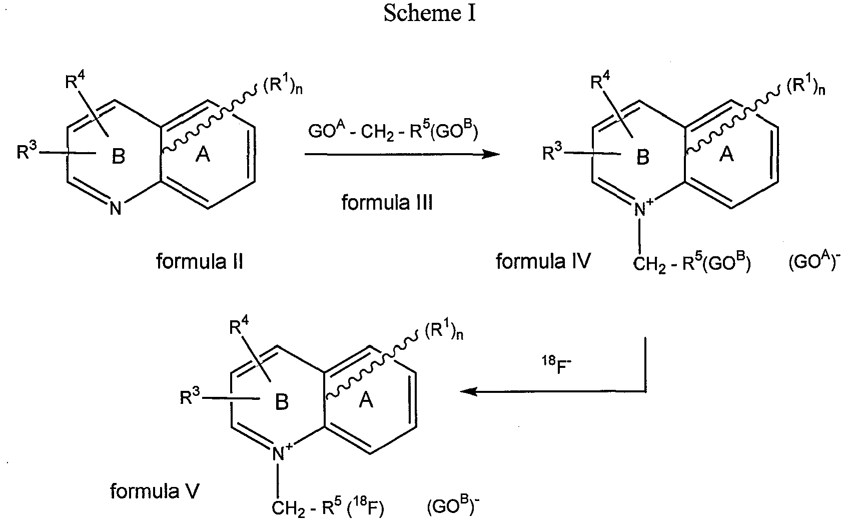

In the first step of the synthesis, the substitution of the GO^{A} group results in the formation of the acridinium or phenanthridinium compound with formula IV with the GO^{B} leaving group in the side chain, and in the second step of the synthesis, the compound with formula IV is reacted with the ¹⁸F⁻ fluoride anion to form the compound with formula V containing the ¹⁸F atom in the side chain attached to the quaternary nitrogen atom.

If required, R¹ groups in a compound with formula II, which could be engaged in side reactions with a compound with formula III, are routinely protected using known protective groups, for example those disclosed in the monograph "Protective Groups in Organic Synthesis" (Theodora W. Greene and Peter G. M. Wuts, 2nd edition, 1991, John Wiley & Sons, Inc.). The R¹ groups in the compound with formula II, that could be engaged in side reactions with the compound with formula III, are in particular primary and secondary amino groups, and optionally hydroxy groups.

Alternatively, the compounds according to the invention are manufactured by the method illustrated in scheme II using a substituted compound with formula II in which R¹, R³ and R⁴ are as defined above for formula I, and the GO^{C}-CH₂-R⁶ compound with formula VI, wherein R⁶ is a ¹⁸F fluorine atom or the CH₂-R⁶ fragment corresponds to the definition of R² in formula I, and the GO^{C} symbol is a leaving group substitutable by a nitrogen atom of the acridine or phenanthridine ring in a nucleophilic substitution reaction. The GO^{C} leaving group is selected from the leaving groups referred to above in the definition of the GO^{A} and GO^{B} groups. According to the method of scheme II, a compound with formula VII containing the ¹⁸F atom in the side chain is manufactured, wherein the R⁶ fragment is the ¹⁸F fluorine atom or the CH₂-R⁶ fragment corresponds to the definition of R² in formula I. Similarly to the reactions according to scheme I, if required, R¹ groups in the compound with formula II, that could be engaged in side reactions with the compound with formula VI, are protected following the standard procedure using the protection groups as above.

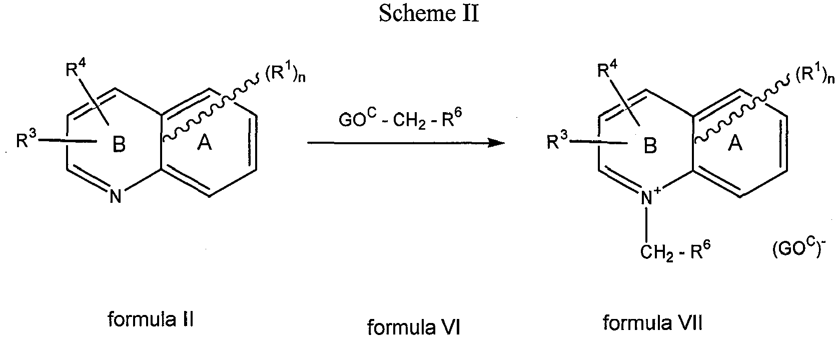

The compound according to the invention is obtained in the form of a quaternary ammonium salt together with a counterion, which is a mononegative anion of a stable organic or inorganic acid, corresponding to the leaving group departing in the last reaction step, in accordance with scheme I or II. The scope of the invention, however, is not limited to such salts, since the mononegative anion being the counterion in the quaternary salt can be replaced with another anion of a stable organic or inorganic acid as required using standard procedures. For example, the quaternary ammonium salt obtained by the method according to scheme I or II is dissolved in a solution of inorganic or organic salt containing the desired mononegative anion, or inorganic or organic salt containing the desired mononegative anion is added to the solution of the quaternary ammonium salt obtained by the method according to scheme I or II. Optionally, the quaternary ammonium salt obtained by the method according to scheme I or II shall be transformed into a salt containing the desired anion using ion exchangers, for example using the method disclosed in the paper entitled "An unusual substitution reaction directed by an intramolecular re-arrangement" (A.D.C. Parenty, L.V. Smith, L. Cronin), Tetrahedron, 61 (2005), pp. 8410-8418, using anion-exchange resin pre-treated with a solution of an alkali metal salt and acid of the desired anion, or recovered by way of elution with an appropriate buffer, in accordance with the procedure presented in the examples below.

Thus, the scope of subject invention covers a radioisotope-labelled compound of quaternary ammonium salt of a polycyclic aromatic amine with formula I, in which formula X⁻ is a mononegative anion of any stable organic or inorganic acid, preferably an anion selected from the group comprising an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid.

Said salts can form hydrates and/or solvates, which hydrates and/or solvates are also included in the scope of the invention.

Preferably, the invention relates to a radioisotope-labelled compound with formula I, in which formula I the wavy line indicates a single bond between the non-nodal carbon atom of a polycyclic aromatic system and the R¹ substituent,
R¹ is a substituent independently selected from hydrogen, halogens, C₁₋₄alkoxy, a nitro group, an amino group optionally having 1 or 2 hydrogen atoms replaced with C₁₋₄alkyl, a chain C₁₋₆carbon group optionally having a halogen or C₁₋₄alkanesulfonic substituent, and a phenyl group optionally having 1-3 substituents independently selected from halogens, a chain C₁₋₄carbon substituent, a halogenated chain C₁₋₄carbon substituent, an amino group optionally having 1-2 atoms of hydrogen replaced with C₁₋₄alkyl,
R² is a chain aliphatic substituent having the -CH₂- fragment as the terminal member of the chain, to which chain a phenyl group is optionally attached optionally having 1-3 substituents selected from halogens and C₁₋₆alkyl, and, if the chain contains at least 2 carbon atoms, in which chain there is optionally a bivalent link between the carbon atoms of the chain, said link selected from the oxygen atom -O- and sulfur atom -S-, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
and the hydrate or solvate thereof.

More preferably, the invention relates to a radioisotope-labelled compound with formula I, in which formula I the wavy line indicates a single bond between the non-nodal carbon atom of a polycyclic aromatic system and the R¹ substituent,
R¹ is a substituent independently selected from hydrogen, halogens, C₁₋₄alkoxy, an amino group optionally having 1-2 hydrogen atoms replaced with C₁₋₄alkyl, a chain C₁₋₄carbon group optionally having a halogen substituent, and a phenyl group optionally having 1-3 substituents independently selected from halogens, a chain C₁₋₄carbon substituent, a halogenated chain C₁₋₄carbon substituent, C₁₋₄alkoxy,
R² is a chain aliphatic substituent having the -CH₂- fragment as the terminal member of the chain, to which chain a phenyl group is optionally attached, optionally having 1-3 substituents selected from halogens and C₁₋₆alkyl, and, if the chain contains at least 2 carbon atoms, in which chain there is optionally a bivalent link between carbon atoms of the chain, said link being the oxygen atom -O-, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
and the hydrate or solvate thereof.

Even more preferably, the compound of the invention is a radioisotope-labelled compound with formula I, in which formula I the wavy line indicates a single bond between the non-nodal carbon atom of a polycyclic aromatic system and the R¹ substituent,
R¹ is a substituent independently selected from hydrogen, halogens, C₁₋₄alkoxy, an amino group optionally having 1-2 hydrogen atoms replaced with C₁₋₂alkyl, a chain C₁₋₄carbon group optionally having a halogen substituent, and a phenyl group optionally having 1-3 substituents independently selected from halogens, a chain C₁₋₄carbon substituent and a halogenated chain C₁₋₄carbon substituent,
R² is a chain aliphatic substituent having the -CH₂- fragment as the terminal member of the chain, to which chain a phenyl group is optionally attached optionally having 1-3 substituents selected from halogens and C₁₋₄alkyl, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
and the hydrate or solvate thereof.

Particularly preferably, the compound of the invention is a radioisotope-labelled compound with formula I, in which formula I the wavy line indicates a single bond between the non-nodal carbon atom of a polycyclic aromatic system and the R¹ substituent,
R¹ is a substituent independently selected from hydrogen, halogens, an amino group optionally having 1-2 hydrogen atoms replaced with C₁₋₂alkyl, a chain C₁₋₄carbon group, and a phenyl group optionally having 1-3 substituents independently selected from a chain C₁₋₄carbon substituent, a halogenated chain C₁₋₄carbon substituent,
R² is a chain aliphatic substituent having the -CH₂- fragment as the terminal member of the chain, to which chain a phenyl group is optionally attached optionally having a halogen substituent, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
and the hydrate or solvate thereof.

The following examples, which include: (i) examples of the manufacturing of intermediates in the manufacturing method of radioisotope-labelled compounds according to the invention, (ii) examples of the manufacturing of standard compounds for radioisotope-labelled compounds according to the invention and (iii) examples of the manufacturing of radioisotope-labelled compounds according to the invention, and (iv) examples of the formulation of a pharmaceutical composition, and (v) examples of the use of said compounds in diagnostic techniques, illustrate in detail the solution according to the invention without limiting the scope thereof. Throughout the examples, DCM stands for dichloromethane and ACN for acetonitrile.

The NMR spectra as quoted, were recorded using the 'zg30' sequence for proton and fluorine spectra and the 'zg_pi_CPD (Bruker 500 MHz) or 's2pul' (Varian 300 MHz) sequence for carbon spectra, with hydrogen's proton decoupling. Proton spectra were calibrated against the TMS present in the sample (samples in chloroform) and against the residual signal of the DMSO solvent of 2.5 ppm (quintet). The external standard CFCl₃ was used for the fluorine spectra measurement.

### Example 1. 2-Fluoroethyl trifluoromethanesulfonate

Trifluoromethanesulfonic acid anhydride (5.000 g; 17.72 mmol) is added to a 100 ml round-bottom single-neck flask with a magnetic dipole and septum. The flask is cooled to a temperature of 0°C using a cooling bath (crushed ice + NaCl aqueous solution). DCM is added using a needle and syringe. A solution of 2-fluoroethanol (1.050 g; 16.39 mmol) and pyridine (1.570 g; 19.85 mmol) in 6 ml DCM is added dropwise over 20 minutes, and the whole is stirred for 2h at room temperature. At the end of the reaction, the mixture is poured into 20 ml of cold water for HPLC. The post-reaction mixture is transferred to a separation funnel and washed three times with water. The organic phase is dried over MgSO₄, and the solvent is then distilled off to dryness on a rotary evaporator to obtain 1.170 g of a pink liquid. Yield36.4%
¹H NMR (CDCl₃, 500 MHz): δ 4.64-4.75 (m, 4H).
¹³C NMR (CDCl₃, 125 MHz): δ 74.7 (d, ²J_{C-F}= 20.25 Hz), 79.9 (d, ¹J_{C-F}=173.88 Hz), 118.6 (q, ¹J_{C-F}= 317.13).
¹⁹F NMR (CDCl₃, 470 MHz): -75.4 (s, 3F), -226.1-(-226.5) (m, 1F). HR-MS C₃H₄F₄O₃S (196.12067 u).

### Example 2. 1,2-Bis(trifluoromethanesulfonyloxy)ethane

Trifluoromethanesulfonic acid anhydride (5.000 g; 17.72 mmol) is added to a 100 ml round-bottom single-neck flask with a magnetic dipole and septum. The reaction flask is cooled to a temperature of 0°C using a cooling bath (crushed ice + NaCl aqueous solution). 12 ml DCM is added using a needle and syringe, after which a solution of ethane-1,2-diol (0.540 g; 8.70 mmol) and pyridine (1.400 g; 17.70 mmol) in 6 ml DCM is added dropwise over 20 minutes, and the whole is stirred for 1.5h at room temperature. At the end of the reaction, the mixture is poured into 20 ml of cold water for HPLC. The post-reaction mixture is transferred to a separation funnel and washed three times with water. The organic phase is dried over MgSO₄, and the solvent is then distilled off to dryness on a rotary evaporator to obtain 2.200 g of a pink liquid. Yield 77.5%.
¹H NMR (CDCl₃, 500 MHz): δ 4.77 (s, 4H).
¹³C NMR (CDCl₃, 125 MHz): δ 71.87, 118.5 (q, ¹J_{C-F}=317.38 Hz).
¹⁹F NMR (CDCl₃, 470 MHz): -74.7 (s, 6F).

### Example 3. 1,6-Bis(trifluoromethanesulfonyloxy)hexane

Trifluoromethanesulfonic acid anhydride (7.000 g; 24.81 mmol) is added to a 100 ml round-bottom single-neck flask with a magnetic dipole and septum. The reaction flask is cooled to a temperature of 0°C using a cooling bath (crushed ice + NaCl aqueous solution). 12 ml DCM is added using a needle and syringe, after which a solution of hexane-1,6-diol (1.400 g; 11.85 mmol) and pyridine (1.450 g; 18.33 mmol) in 50 ml DCM is added dropwise over 20 minutes. The whole is stirred for 3h at room temperature. At the end of the reaction, the mixture is poured into 20 ml of cold water for HPLC. The post-reaction mixture is transferred to a separation funnel and washed three times with water. The organic phase is dried over MgSO₄, and the solvent is then distilled off to dryness on a rotary evaporator to obtain 2.000 g of the product. Yield 44.2%.
¹H NMR (CDCl₃, 500 MHz): δ 1,51 (quint, 4H, ³J_{H-H}=4 Hz), 1.80-1.90 (m, 4H), 4.55 (t, ³J_{H-H}=6.5 Hz, 4H).
¹³C NMR (CDCl₃, 125 MHz): δ 24.5, 28.9, 77.2, 118.6 (q, ¹J_{C-F}=317.25 Hz).
¹⁹F NMR (CDCl₃, 282 MHz): -75.0.
HR-MS C₈H₁₂F₆O₆S₂ (382.29770 u).

### Example 4. 1,3-Bis(trifluoromethanesulfonyloxy)propane

Trifluoromethanesulfonic acid anhydride (2.104 g; 7.46 mmol) is added to a 100 ml round-bottom single-neck flask with a magnetic dipole and septum. The reaction flask is cooled to a temperature of 0°C using a cooling bath (crushed ice + NaCl aqueous solution). 12 ml DCM is added using a needle and syringe, after which a solution of propane-1,3-diol (0.261 g; 3.43 mmol) and pyridine (0.620 g; 7.84 mmol) in 6 ml DCM is added dropwise over 20 minutes, and the whole is stirred for 1.5h at room temperature. At the end of the reaction, the mixture is poured into 20 ml of cold water for HPLC, transferred to a separation funnel and washed three times with water. The organic phase is dried over MgSO₄, and the solvent is then distilled off to dryness on a rotary evaporator to obtain 0.938 g of the title compound in the form of a pink liquid. Yield 80.38%.
¹H NMR (CDCl₃, 500 MHz): δ 2.37 (q, ³J_{H-H}=5.5 Hz, 2H), 4.68 (t, ³J_{H-H}=6.0 Hz, 4H).
¹³C NMR (CDCl₃, 125 MHz): δ 29.3, 71.5, 118.6 (q, ¹J_{C-F}=319.60 Hz).
¹⁹F NMR (CDCl₃, 470 MHz): -74.6 (s, 6F).

### Example 5. 1,16-Bis(trifluoromethanesulfonyloxy)hexadecane

Trifluoromethanesulfonic acid anhydride (2.50 g; 8.86 mmol) and hexadecane-1,16-diol (0.252 g; 0.98 mmol) are added to a 100 ml round-bottom single-neck flask with a magnetic dipole and septum. The reaction is carried out at room temperature for 4h. At the end of the reaction, the mixture is poured into 20 ml DCM and cold water for HPLC. The post-reaction mixture is transferred to a separation funnel and washed three times with water. The phase is dried over MgSO₄, and the solvent is then distilled off to dryness on a rotary evaporator to obtain 0.301 g of the product. Yield 17.4%.
¹H NMR (CDCl₃, 500 MHz): δ 1.20 - 1.30 m (20 H), 1.41 (quint, 4H, ³J_{H-H}=4 Hz), 1.82 (quint, ³J_{H-H}= 4 Hz, 4H), 4.54 (t, ³J_{H-H}=6.5 Hz, 4H).
¹³C NMR (CDCl₃, 125 MHz): δ 25.0, 28.8, 29.2, 29.3, 29.4, 29.6, 29.6, 77.8, 118.6 (q, ¹J_{C-F}=317.25 Hz).
¹⁹F NMR (CDCl₃, 470 MHz): -74.9.

### Example 6. 2-(4-Fluorophenyl)ethyl trifluoromethanesulfonate

Trifluoromethanesulfonic acid anhydride (2.000 g; 7.09 mmol) is added to a 50 ml round-bottom single-neck flask with a magnetic dipole and septum. The solution is cooled to a temperature of 0°C using a cooling bath (ice + NaCl), 2-(4-fluorophenyl)ethanol (0.921 g; 6.57 mmol) is added dropwise to the cooled solution over about 10 minutes, and the contents of the flask are stirred for 4h at room temperature. At the end of the reaction, the mixture is poured into 20 ml DCM and cold water for HPLC. The reaction mixture is transferred to a separation funnel and washed repeatedly with water to remove the acid. The phase is dried over MgSO₄, and the solvent is then distilled off on a rotary evaporator to obtain 0.999 g of the product. Yield 55.8%.
¹H NMR (CDCl₃, 500 MHz): δ 3.10 (t, ³J_{H-H}=7,0 Hz,), 4.66 (td, ³J_{H-H}=7,0 Hz, ⁴J_{H-F}=0.5 Hz, 2H), 7.03 (dd, ³J_{H-F}= 9.0 Hz, ⁴J_{H-H}= 5.0 Hz, 2H), 7.16-7.20 (m, ³J_{H-H}=7.0 Hz, 2H).
¹³C NMR (CDCl₃, 125 MHz): δ 35.24, 77.55, 116.2 (d, ²J_{C-F}= 21.5 Hz), 118.6 (q, ¹J_{C-F}= 317.13), 130.8090 (d, ³J_{C-F}= 8.0 Hz), 130.8095 (d, ⁴J_{C-F}= 3.4 Hz), 162.5 (d, ¹J_{C-F}=246.2 Hz).
¹⁹F NMR (CDCl₃, 282 MHz): -74.8 (s, 3F), -115.0 (tt, ³J_{F-H}=8.8 Hz, ⁴J_{F-H}=5.0 Hz, 1F).

### Example 7. 5-(2-Fluoroethyl)phenanthridinium p-toluenesulfonate

Phenanthridine (0.410 g; 2.29 mmol) is added to a 100 ml round-bottom single-neck flask in an inert gas atmosphere, after which 1 ml of toluene is added three times using a needle and syringe, which is then distilled off to dryness. Phenanthridine dissolved in DMF is added dropwise to 2-fluoroethyl tosylate (1.000 g; 4.58 mmol) in DMF, contained in a 100 ml round-bottom single-neck flask equipped with a magnetic dipole and mounted on a magnetic stirrer. The reaction is carried out in an inert gas atmosphere for 168h at 105°C. At the end of the reaction, the yellow to brown solution is concentrated on a rotary evaporator to remove DMF. The residue is cooled to room temperature and dissolved in cold acetone (about 4 ml). Diethyl ether (about 60 ml) is added, and this is cooled in a refrigerator. The precipitated product is filtered off. 0.20 g of the title compound is obtained (22.0% yield).

¹H NMR ((CD₃)₂SO, 500 MHz): δ 5.09 (td, ²J_{H-F}=47 Hz, ³J_{H-H}=4.5 Hz, 2H), 5.53 (td, ³J_{H-F}=26 Hz, ³J_{H-H}=4.5 Hz, 2H), 7.94-8.04 (m, 4H), 8.10-8.17 (m, 4H), 8.20-8.30 (m, 4H), 8.41-8.45 (m, 1H), 8.54 (dd, ³J_{H-H} = 8 Hz, ⁴J_{H-H} = 0.5 Hz, 1H), 8.62-8.69 (m, 2H), 9.00 - 9.06 (m, 2H), 9.16 (d, ³J_{H-H}= 8 Hz, 1H), 9.21 (dd, ³J_{H-H} = 8 Hz, ⁴J_{H-H}=2 Hz), 10.37 (s, 1H).

¹⁹F NMR ((CD₃)₂SO, 470 MHz): δ -221.1 (tt, ²J_{F-H}=47.1 Hz, ³J_{H-H}=25.9 Hz, 1F). HR-MS C₇H₇S₁O₃⁻ (171.01104 u), found: 171.01122 u, C₁₅H₁₃N₁F₁⁺ (226.10265 u), found: 226.10250 u.

### Example 8. 6-Phenyl-5-(2-fluoroethyl)phenanthridinium trifluoromethanesulfonate

2-Fluoroethyl trifluoromethanesulfonate (0.290 g; 1.48 mmol) is added to a 50 ml round-bottom single-neck flask with a magnetic dipole. 6 ml DCM is added using a needle and syringe. The solution is cooled to a temperature of -56°C using a cooling bath (liquid nitrogen + ethyl acetate). A solution of 6-phenylphenanthridine (0.210 g; 0.82 mmol) in 6 ml DCM is added dropwise to the cooled solution over about 20 minutes. The contents of the flask are stirred for 216h. At the end of the reaction, cold Et₂O is added to the reaction mixture. The mixture is left in the refrigerator for 30 min, after which the product is filtered under reduced pressure. 0.200 g of the title compound is obtained (53.9% yield).
¹H NMR ((CD₃)₂SO, 500 MHz): δ 4.95 (dt, ²J_{H-F}=46.5 Hz, ³J_{H-H}=5.0 Hz, 2H), 5.30 (dbs, ³J_{H-F}=23.5 Hz, 2H), 7.56 (d, ³J_{H-H}=8 Hz, 1H), 7.75-7.88 (m, 5H), 7.96 (t, ³J_{H-H}=7.5 Hz, 1H), 8.15-8.23 (m, 2H), 8.48 (ddd, ³J_{H-H}=8.5 Hz, ³J_{H-H}=7.0 Hz, ⁴J_{H-H}=1.5 Hz, 1H), 8.75 (d, ³J_{H-H}=9 Hz, 1H), 9.28 (d, ³J_{H-H}=8 Hz, 1H), 9.32 (dd, ³J_{H-H}=8.5 Hz, ⁴J_{H-H}=1.5 Hz, 1H).
¹³C NMR ((CD₃)₂SO, 125 MHz): δ 54.1 (d, ²J_{C-F}= 21.00 Hz), 81.3 (d, ¹J_{C-F}=170.00 Hz), 121.1, 123.3, 124.8, 125.5, 126.0, 128.9, 129.2, 130.4, 130.6, 130.9, 131.4, 132.3, 132.9, 134.2, 134.7, 137.8, 165.3.
¹⁹F NMR ((CD₃)₂SO, 470 MHz): δ -78.1 (s, 3F), -220.4 - -220.8 (tt, ²J_{F-H}=49.0 Hz, ³J_{H-H}=22.1 Hz, 1F).
HR-MS C₁O₃F₃S₁⁻ (148.95148 u), found: 148.95106 u, C₂₁H₁₇FN⁺ (302.36423 u).

### Example 9. 10-(2-Fluoroethyl)acridinium trifluoromethanesulfonate

2-Fluoroethyl trifluoromethanesulfonate (0.270 g; 1.38 mmol) is added to a 50 ml round-bottom single-neck flask with a magnetic dipole. 6 ml DCM is added using a needle and syringe. The solution is cooled to a temperature of -56°C using a cooling bath (liquid nitrogen + ethyl acetate). A solution of acridine (0.220 g; 1.23 mmol) in 6 ml DCM is added dropwise to the cooled solution over about 20 minutes, after which the contents of the flask are stirred for 72 h. At the end of the reaction, 20 ml of cold Et₂O is added to the reaction mixture, the mixture is left in the refrigerator for 30 min, and then the product is filtered off under reduced pressure. 0.200 g of the title compound is obtained. Yield 43.4%.
¹H NMR ((CD₃)₂SO, 500 MHz): δ 5.15 (dt, ²J_{H-F}=47 Hz, ³J_{H-H}=4.5 Hz, 2H), 5.91 (dt, ³J_{H-F}=25.5 Hz, J= 4.5 Hz, 2H), 8.06 (dd, ³J_{H-H}=8.5 Hz, ³J_{H-H}=7.0 Hz, 2H), 8.48 (ddd, ³J_{H-H}=9.5 Hz, ³J_{H-H}=7.0 Hz, ⁴J_{H-H}=1.5 Hz, 2H), 8.67 (dd, ³J_{H-H}=8 Hz, ⁴J_{H-H}=1.5 Hz, 2H), 8.80 (d, ³J_{H-H}=9.5 Hz, 2H), 10.26 (s, 1H).
¹³C NMR ((CD₃)₂SO, 125 MHz): δ 49.9 (d, ²J_{C-F}=19.12 Hz), 82.3 (d, ¹J_{C-F}=168.88 Hz), 118.9, 126.4, 127.8, 132.0, 139.4, 141.5, 152.1.
¹⁹F NMR ((CD₃)₂SO, 470 MHz): δ -78.1 (s, 3F), -221.5 (tt, ²J_{F-H}=47 Hz, ²J_{F-H}=25.5 Hz, 1F).
HR-MS C₁O₃F₃S₁⁻ (148.95148 u), C₁₅H₁₃N₁F₁⁺(226.10265 u), found: 226.10250 u.

### Example 10. 3,6-Bis(dimethylamino)-10-(2-fluoroethyl)acridinium trifluoromethanesulfonate

2-Fluoroethyl trifluoromethanesulfonate (0.218 g; 1.11 mmol) is added to a 50 ml round-bottom single-neck flask with a magnetic dipole. 10 ml DCM is added using a needle and syringe. The solution is cooled to a temperature of -56°C using a cooling bath (liquid nitrogen + ethyl acetate). A solution of Acridinium Orange (0.137 g; 0.52 mmol) in 10 ml DCM is added dropwise to the cooled solution over about 20 minutes, after which the contents of the flask are stirred for 72h. At the end of the reaction, 20 ml of cold Et₂O is added to the reaction mixture, the mixture is left in the refrigerator for 30 min, and then the product is filtered off under reduced pressure. 0.104 g of the title compound is obtained (57.5% yield).
¹H NMR ((CD₃)₂SO, 500 MHz): δ 3.19 (s, 12H), 4.90-5.10 (m, 4H), 6.54 (s, 2H), 7.10 (d, ³J_{H-H}=9 Hz, 2H), 7.73 (d, ³J_{H-H}=9 Hz, 2H), 8.56 (s, 1H).
¹³C NMR ((CD₃)₂SO, 125 MHz): δ 40.2, 46.8 (d, ²J_{C-F}=19.9 Hz), 81.6 (d, ¹J_{C-F}=167.6 Hz), 92.9, 114.0, 116.2, 120.7 (q, ¹J_{C-F}=320 Hz), 132.8, 142.6, 143.0, 155.2.
¹⁹F NMR ((CD₃)₂SO, 282 MHz): δ -77.71 (s, 3F), -221.3 (tt, ²J_{F-H}=49.4 Hz, ³J_{H-H}=25.1 Hz, 1F).
HR-MS C₃F₃S₁O₃⁻ (148.95148 u), found: 148.95106 u, C₁₉H₂₃FN₃ (312.40387 u).

### Example 11. 5-(2-Trifluoromethylsulfonyloxyethyl)phenanthridinium trifluoromethanesulfonate

1,2-Bis(trifluoromethanesulfonyloxy)ethane (0.310 g, 0.95 mmol) is added to a 50 ml round-bottom single-neck flask with a magnetic dipole. 6 ml DCM is added using a needle and syringe. The solution is cooled to a temperature of -56°C using a cooling bath (liquid nitrogen + ethyl acetate). A solution of phenanthridine (0.170 g, 0.95 mmol) in 10 ml DCM is added dropwise to the cooled solution over about 20 minutes, after which the contents of the flask are stirred for 48h. At the end of the reaction, 20 ml of cold Et₂O is added to the reaction mixture, the mixture is left in the refrigerator for 30 min, and then the product is filtered off under reduced pressure. 0.160 g of the title compound is obtained (33.3% yield).
¹H NMR ((CD₃)₂SO, 500 MHz): δ 4.88 (t, ³J_{H-H} = 4.5 Hz, 2H), 5.48 (t, ³J_{H-H}=4.5 Hz, 2H), 8.10-8.25 (m, 3H), 8.46 (ddd, ³J_{H-H}=8.0 Hz, ³J_{H-H}=7.0 Hz, ⁴J_{H-H}=1.0 Hz, 2H), 8.66 (dd, ³J_{H-H}=8.0 Hz, ⁴J_{H-H}=1.0 Hz, 2H), 9.19 (d, ³J_{H-H}=8.5 Hz, 1H), 9.24 (dd, ³J_{H-H}=8.5 Hz, ⁴J_{H-H}=2.0 Hz, 1H), 10.23 (s, 1H).
¹³C NMR ((CD₃)₂SO, 125 MHz); δ 56.9, 73.1, 120.0, 120.7 (q, ¹J_{C-F}= 320.25), 123.2, 123.5, 125.1, 126.0, 130.4, 130.5, 132.0, 133.1, 133.6, 134.8, 138.6, 156.6. ¹⁹F NMR ((CD₃)₂SO, 470 MHz); δ -78.1 (s, 3F).
HR-MS C₁F₃S₁O₃⁻ (148.95148 u), found: 148.95147 u, C₁₆H₁₃N₁S₁O₃F₃⁺ (356.05628 u), found: 356.05616 u.

### Example 12. 6-Phenyl-5-(2-trifluoromethylsulfonyloxyethyl)phenanthridinium trifluoromethanesulfonate

1,2-Bis(trifluoromethanesulfonyloxy)ethane (0.404 g; 1.24 mmol) is added to a 50 ml round-bottom single-neck flask with a magnetic dipole. 6 ml DCM is added using a needle and syringe. The solution is cooled to a temperature of -56°C using a cooling bath (liquid nitrogen + ethyl acetate), 6-phenylphenanthridine solution (0.170 g; 0.67 mmol) in 6 ml DCM is added dropwise over about 20 minutes to the cooled solution, and the contents of the flask are then stirred for 96h. At the end of the reaction, the mixture is concentrated to 8 ml, 40 ml of cold Et₂O is added, and this is left in the refrigerator for 30 minutes. The product is filtered under reduced pressure to obtain 0.200 g of the title compound (51.7% yield).
¹H NMR ((CD₃)₂SO, 500 MHz): δ 4.70 (t, 2H, ³J_{H-H}=4.5 Hz), 5.26 (bs, 2H), 7.58 (d, 1H, ³J_{H-H}=8.5 Hz), 7.72-7.88 (m, 5H), 7.98 (t. 1H, J=7.5 Hz), 8.15-8.25 (m, 2H), 8.42 (t, 1H, J = 8.0 Hz), 8.65 (d, 1H, ³J_{H-H}=9.5 Hz), 9.29 (d, 1H, ³J_{H-H}=8.5 Hz), 9.34 (d, 1H, ³J_{H-H}=9.5 Hz).
¹⁹F NMR ((CD₃)₂SO, 470 MHz): δ -78.1 (s, 3F).
MS-HR C₁O₃F₃S₁⁻ (148.95148), found: 148.95106, C₂₂H₁₇F₃NO₃S (432.43484 u)
found: 432.08751 u.

### Example 13. 10-(2-Trifluoromethylesulfonyloxyethyl)acridinium trifluoromethanesulfonate

1,2-Bis(trifluoromethanesulfonyloxy)ethane (0.29 g, 0.89 mmol) is added to a 50 ml round-bottom single-neck flask with a magnetic dipole. 6 ml DCM is added using a needle and syringe. The solution is cooled to a temperature of -56°C using a cooling bath (liquid nitrogen + ethyl acetate), acridine solution (0.120 g; 0.67 mmol) in 6 ml DCM is added dropwise over about 20 minutes to the cooled solution, and the contents of the flask are then stirred for 48h. At the end of the reaction, the mixture is concentrated to 4 ml, 20 ml of cold Et₂O is added, and this is left in the refrigerator for 30 minutes. The product is filtered under reduced pressure to obtain 0.03 g of the title compound. Yield 8.86%.
¹H NMR ((CD₃)₂SO, 500 MHz): δ 5.39 (t, ³J_{H-H}=5.0 Hz, 2H), 6.02 (t, ³J_{H-H}=5.0 Hz, 2H), 8.00-8.10 (dd, ³J_{H-H}=8.0 Hz, ³J_{H-H}=7.0 Hz, 2H), 8.15 (ddd, ³J_{H-H}=9.0 Hz, ³J_{H-H}=7.0 Hz, ⁴J_{H-H}=1.5 Hz, 2H,), 8.32 (d, ³J_{H-H}=9.5 Hz, 2H), 8.47 (dd, ³J_{H-H}=8.5 Hz, ⁴J_{H-H}=1 Hz, 2H), 9.99 (s, 1H).
¹⁹F FMR ((CD₃)₂SO, 470 MHz): δ -78.1 (s, 3F).
MS-HR C₃F₃S₁O₃⁻ (148.95148), found: 148.95106, C₁₆H₁₃F₃NO₃S⁺ (356.338981 u).

### Example 14. 3,6-Bisdimethylamino-10-(2-trifluoromethylesulfonyloxyethyl)-acridinium trifluoromethanesulfonate

1,2-Bis(trifluoromethanesulfonyloxy)ethane (0.355 g, 1.09 mmol) is added to a 50 ml round-bottom single-neck flask with a magnetic dipole. 6 ml DCM is added using a needle and syringe. The solution is cooled to a temperature of -56°C using a cooling bath (liquid nitrogen + ethyl acetate), and a solution of Acridinium Orange (0.207 g, 0.78 mmol) in 6 ml DCM is added dropwise to the cooled solution over about 20 minutes. The contents of the flask are stirred for 96h and, at the end of the reaction, concentrated to 4 ml, and 20 ml of cold Et₂O is added. The mixture is left in the refrigerator for 30 min, after which the product is filtered under reduced pressure to obtain 0.310 g of the title compound. Yield 67.2%.
¹H NMR ((CD₃)₂SO, 500 MHz): δ 3.27 (s, 12H), 4.85 (t, ³J_{H-H}=4.5 Hz, 2H), 5.15 (bs, 2H), 6.63 (s, 2H), 7.25 (d, ³J_{H-H}=9.0 Hz, 2H), 7.89 (d, ³J_{H-H}=7.5 Hz, 2H), 8.76 (s, 1H).
¹³C NMR ((CD₃)₂SO, 125 MHz): δ 40.4, 57.8, 73.2, 93.1, 114.2, 116.4, 120.7 (q, ¹J_{C-F}=320 Hz), 133.0, 143.0, 155.5.
¹⁹F NMR ((CD₃)₂SO, 470 MHz): δ -78.1 (s, 3F).

### Example 15. 5-(3-Fluoropropropyl)phenanthridinium trifluoromethanesulfonate

The title compound is obtained by following the procedure described in example 7, but using 3-fluoropropropyl trifluoromethanesulfonate (0.229 g, 1.09 mmol) instead of 2-fluoroethyl trifluoromethanesulfonate and phenanthridine (0.230 g, 1.28 mmol). Yield 71.0%.
¹H NMR ((CD₃)₂SO, 500 MHz): δ 2.46-2.58 (m, 2H), δ 4.73 (dt, ²J_{H-F}=47 Hz, ³J_{H-H}=5.5 Hz, 2H), 5.24 (t, ³J_{H-H}=7 Hz, 2H,), 8.09-8.14 (m, 2H), 8.14-8.19 (m, 1H), 8.37-8.43 (m, 1H), 8.59 (dd, ³J_{H-H}=8 HZ, ⁴J_{H-H}=1 Hz, 1H), 8.63 (d, ³J_{H-H}=8 Hz, 1H), 9.12 (d, ³J_{H-H}=8 Hz, 1H), 9.18 (dd, ³J_{H-H}=8 Hz, ⁴J_{H-H}=1 Hz,1H), 10.37 (s, 1H).
¹³C NMR ((CD₃)₂SO, 125 MHz): δ 29.8 (d, ¹J_{C-F}=19.375 Hz), δ 55.0 (d, ¹J_{C-F}=4.5 Hz), 81.5 (d, ¹J_{C-F}=161.0 Hz), 119.8, 120.7 (q, ¹J_{C-F}=320.25 Hz), 123.1, 123.7, 125.1, 125.9, 130.3, 130.4, 132.1, 132.8, 133.1, 134.4, 138.1, 155.9.
¹⁹F NMR ((CD₃)₂SO, 470 MHz): δ -219.7 - -220.1 (m, 1F), -77.72 (s, 3F).

### Example 16. 5-[2-(4-Fluorophenyl)ethyl]phenanthridinium trifluoromethanesulfonate

The title compound is obtained by following the procedure described in example 7, but using 2-(4-fluorophenyl)ethyl trifluoromethanesulfonate (0.174 g, 0.64 mmol) instead of 2-fluoroethyl trifluoromethanesulfonate and phenanthridine (0.147 g, 0.82 mmol). 0.198 g of the product is obtained (53.7% yield).
¹H NMR ((CD₃)₂SO, 500 MHz): δ 3.41 (³J_{H-H}=7.5 Hz. 2H). 5,33 (t. ³J_{H-H}=7,5 Hz. 2H.), 7,09-7,14 (m. 2H). 7,27-7,33 (m, 2H), 8.10 (ddd, ³J_{H-H}=8.0 Hz, ³J_{H-H}=7.5 Hz, ⁴J_{H-H}=1.0 Hz, 1H), 8.14 (ddd, ³J_{H-H}=8.0 Hz, ³J_{H-H}=7.5 Hz, ⁴J_{H-H}=1.5 Hz, 1H), 8.19 (ddd, ³J_{H-H}=9.0 Hz, ³J_{H-H}=7.5 Hz, ⁴J_{H-H}=1.5 Hz, 1H), 8.41 (ddd, ³J_{H-H}=8.5 Hz, ³J_{H-H}=7.0 Hz, ⁴J_{H-H}=1.5 Hz, 1H), 8.48 (dd, ³J_{H-H}=8.0 Hz, ⁴J_{H-H}=1.0 Hz, 1H), 8.74 (dd, ³J_{H-H}=8.5 Hz, ⁴J_{H-H}=1.0 Hz, 1H), 9.15 (d, ³J_{H-H}=8.5 Hz, 1H), 9.21 (dd, ³J_{H-H}=8 Hz, ⁴J_{H-H}=1.5 Hz, 1H), 10.13 (s, 1H).
¹³C NMR ((CD₃)₂SO, 125 MHz): δ 34.1, 58.5, 115.4 (d, ²J_{C-F}=21.3 Hz), 120.1, 120.7 (q, ¹J_{C-F}=320.25 Hz), 123.2, 123.4, 125.1, 125.8, 130.5, 130.4, 131.0 (d, ³J_{C-F}=8.2 Hz), 132.2, 132.6 (d, ⁴J_{C-F}=3.1 Hz), 132.2 132.7, 132.9, 134.4, 138.2, 155.5, 161.3 (d, ¹J_{C-F}=243.2 Hz).
¹⁹F NMR ((CD₃)₂SO, 470 MHz): δ -77.72 (s, 3F), -115.6 - -115.5 (m, 1F).

### Example 17. 10-(2-Iodoethyl)acridinium iodide

1.2-Diiodoethane (1.033 g, 3.66 mmol) is added to a 50 ml round-bottom single-neck flask with a magnetic dipole. Acridine (0.406 g, 2.26 mmol) dissolved in 10 ml toluene is added using a needle and syringe, and the reaction is carried out at solvent boiling point for 10h. The mixture is cooled, the precipitate formed is filtered off and washed with diethyl ether. 0.433 g of a compound with 33% purity (according to HPLC) is obtained.
1H NMR ((CD₃)₂SO, 500 MHz): δ 3.79 (t, ³J_{H-H}=7.5 Hz, 2H), 5.76 (t, ³J_{H-H}=7.5 Hz, 2H), 8.03 (dd, ³J_{H-H}=8.0 Hz, ³J_{H-H}=7.0 Hz, 2H), 8.48 (ddd, ³J_{H-H}=8.5 Hz, ³J_{H-H}=7.5 Hz, ⁴J_{H-H}=1.5 Hz, 2H), 8.63 (dd, ³J_{H-H}=8.5 Hz, ⁴J_{H-H}=1.5 Hz, 2H), 8.69 (d, ³J_{H-H}=9.5 Hz, 2H) 10.20 (s, 1H).

### Example 18. 5-(16-Trifluoromethylsulfonyloxyhexadecyl)phenanthridinium trifluoromethanesulfonate

1,16-Bis(trifluoromethanesulfonyloxy)hexadecane (0.145 g, 0.28 mmol) is added to a 50 ml round-bottom single-neck flask with a magnetic dipole. 15 ml DCM is added using a needle and syringe. A solution of phenanthridinium (0.033 g, 0.18 mmol) in 15 ml DCM is added dropwise to the solution over about 20 minutes, after which the contents of the flask are stirred for 96h. At the end of the reaction, DCM is evaporated, and the residue is washed with diethyl ether. 0.038 g of the title compound is obtained. Yield 19.5%.

¹H NMR ((CD₃)₂SO, 500 MHz): δ 1.1-1.4 (m, 20H), 1.45 (bs, 2H), 2.06 (bs, 2H), 4.5-4.7 (bs, 2H), 5.08 (t, 2H, ³J_{H-H}=7.5 Hz), 8.07-8.17 (m, 3H), 8.40 (ddd ³J_{H-H}=8.5 Hz, ³J_{H-H}=7.5 Hz, ³JH-H=1.5 Hz, 1H), 8.58 (d, ³J_{H-H}=8.0 Hz, 1H), 8.64 (d, ³J_{H-H}=8.5 Hz, 1H), 9.14 (d, ³J_{H-H}=8.0 Hz, 1H), 9.19 (dd, ³J_{H-H}=8.0 Hz, ⁴J_{H-H}=1.5 Hz, 1H), 10.32 (s, 1H).

¹³C NMR ((CD₃)₂SO, 125 MHz): δ 24.7, 25.5, 25.7, 25.9, 28.5, 28.6, 28.8, 28.86, 28.91, 28.96, 29.00, 29.02, 29.06, 29.09, 60.7, 76.2, 119.4, 120.7 (q, ¹J_{C-F}=320.25), 123.2, 123.7, 125.0, 125.9, 130.3, 130.4, 132.1, 132.8, 133.1, 134.4, 138.0, 155.3.

### Example 19. 6-[4-(Chloromethyl)phenyl]-5-(2-fluoroethyl)phenanthridinium trifluoromethanesulfonate

2-Fluoroethyl trifluoromethanesulfonate (0.091 g, 0.46 mmol) is added to a 50 ml round-bottom single-neck flask with a magnetic dipole. 6 ml DCM is added using a needle and syringe. A solution of 6-[4-(chloromethyl)phenyl]phenanthridine (0.071 g, 0.23 mmol) in 6 ml DCM is added dropwise to the cooled solution over about 20 minutes. The contents of the flask are mixed for 70.5h. At the end of the reaction, cold Et₂O (40 ml) is added to the reaction mixture. The mixture is left in the refrigerator for 30 min, after which the product is filtered under reduced pressure. 0.019 g of the title compound is obtained (15.6% yield).
¹H NMR ((CD₃)₂SO, 500 MHz): δ 4.93 (dt, ²J_{H-F}=47.0 Hz, ³J_{H-H}= 4.5 Hz, 2H), 4.99 (s, 2H), 5.27 (bd, ³J_{H-F}=23.5 Hz), 7.54 (dd, ³J_{H-H}=8.5 Hz, ⁴J_{H-H}=1.0 Hz, 1H), 7.80 (d, ³J_{H-H}=8.0 Hz, 2H), 7.87 (d, ³J_{H-H}=8.0 Hz, 2H), 7.97 (ddd, ³J_{H-H}=8.5 Hz, ³J_{H-H}=7.0 Hz, ⁴J_{H-H}=1.0 Hz, 1H), 8.17-8.23 (m, 2H), 8.41 (ddd, ³J_{H-H} = 8.0 Hz, ³J_{H-H}=7.0 Hz, ⁴J_{H-H}=1.5 Hz, 1H), 8.74 (d, ³J_{H-H}=9.5 Hz, 1H), 9.28 (d, ³J_{H-H}=8.5 Hz, 1H), 9.33 (dd, ³J_{H-H}=7.0 Hz, ⁴J_{H-H}=2.0 Hz, 1H).
¹³C NMR ((CD₃)₂SO, 125 MHz): δ 45.3, 54.2 (d, ²J_{C-F}=21.3 Hz), 81.3 (d, ²J_{C-F}=170.7 Hz), 121.1, 120.7 (q, ¹J_{C-F}=320.25 Hz), 120.8, 123.3, 124.9, 125.4 126.0, 129.4, 130.5, 130.6, 130.7, 132.3, 132.8, 134.1, 134.7, 137.8, 140.9, 164.9. ¹⁹F NMR ((CD₃)₂SO, 282 MHz): δ -77.7 (s, 3F), -220.2 (tt, 46.9, 24.0, 1F). HR-MS C₁O₃F₃S₁⁻ (148.95148), found: 148.95106, C₂₂H₁₈FClN⁺ (350.11063), found: 350.11040.

### Example 20. 9-Chloro-10-[2-(4-fluorophenyl)ethyl]acridinium trifluoromethanesulfonate

2-(4-Fluorophenyl)ethyl trifluoromethanesulfonate (0.068 g, 0.25 mmol) is added to a 50 ml round-bottom single-neck flask with a magnetic dipole. 6 ml DCM is added using a needle and syringe. A solution of 9-chloroacridine (0.057 g, 0.27 mmol) in 6 ml DCM is added dropwise to the solution over about 20 minutes. The contents of the flask are mixed for 19h. At the end of the reaction, cold Et₂O (40 ml) is added to the reaction mixture. The mixture is left in the refrigerator for 30 min, after which the product is filtered under reduced pressure. 0.001 g of the title compound is obtained (0.6% yield).

HR-MS C₁O₃F₃S₁⁻ (148.95148), found: 148.95106, C₂₁H₁₆FClN⁺ (336.09498), found: 336.09481.

### Example 21. 6-(4-Butylphenyl)-5-[2-(4-fluorophenyl)ethyl]phenanthridinium trifluoromethanesulfonate

The title compound is obtained by following the procedure described in example 20, but using 6-(4-butylphenyl)phenanthridine (0.103 g, 0.33 mmol) instead of 9-chloroacridine and 2-(4-fluorophenyl)ethyl trifluoromethanesulfonate (0.1955 g, 0.72 mmol). 0.032 g of the product is obtained (16.5% yield).
¹H NMR ((CD₃)₂SO, 500 MHz): δ 0.98 (t, ³J_{H-H}=7.5 Hz, 3H), 1.39 (sext, ³J_{H-H}=7.5 Hz, 2H), 1.71 (quint, ³J_{H-H}=7.5 Hz, 2H), 2.82 (t, ³J_{H-H}=7.5 Hz, 3H), 3.26 (t, ³J_{H-H}=7.5 Hz, 2H), 4.94 (bs, 2H), 6.97-7.10 (m, 4H), 7.60-7.75 (m, 5H), 7.97 (t, ³J_{H-H}=8.0 Hz, 1H), 8.20-8.30 (m, 2H), 8.40 (td, ³J_{H-H}=8.0 Hz, ⁴J_{H-H} = 1.0 Hz, 1H), 8.90 (d, ³J_{H-H}=8.5 Hz, 1H), 9.26 (d, ³J_{H-H}=10.00 Hz, 1H), 9.33 (dd, ³J_{H-H}=8.5 Hz, ⁴J_{H-H}=1.0 Hz, 1H).
¹³C NMR ((CD₃)₂SO, 125 MHz): δ 13.8, 21.6, 33.0, 33.5, 34.7, 55.3, 115.3 (d, ²J_{C-F}=21.3, 21.00 Hz), 121.0, 120.7 (q, ¹J_{C-F}=320.25 Hz), 123.2, 124.9, 125.6 125.9, 128.4, 129.2, 133.0, 133.5, 130.5 (d, ³J_{C-F}=8.2 Hz), 132.5, 132.7, 132.7 (d, ⁴J_{C-F}=2.9 Hz), 133.8, 134.5, 137.5, 161.3 (d, ¹J_{C-F}=243.5 Hz), 164.6.
¹⁹F NMR ((CD₃)₂SO, 470 MHz); δ -77.7 (s, 3F), -115.5 - -115.4 (m, 1F).

### Example 22. 6-Phenyl-5-(3-fluoropropyl)phenanthridinium trifluoromethanesulfonate

3-Fluoropropropyl trifluoromethanesulfonate (0.159 g, 0.76 mmol) is added to a 50 ml round-bottom single-neck flask with a magnetic dipole. 6 ml DCM is added using a needle and syringe. The solution is cooled to a temperature of -56°C using a cooling bath (liquid nitrogen + ethyl acetate). A solution of 6-phenylphenanthridine (0.149 g, 0.58 mmol) in 6 ml DCM is added dropwise to the cooled solution over about 20 minutes. The contents of the flask are mixed for 96h. At the end of the reaction, the product is concentrated on an evaporator, cold Et₂O is added, and the mixture is left in the refrigerator for 30 minutes. The precipitated product is filtered off under reduced pressure. 0.136 g of the title compound is obtained (50.0% yield).
¹H NMR ((CD₃)₂SO, 500 MHz): δ 2.36 (dq, ³J_{H-F}=27.0 Hz, ³J_{H-H}= 5.5 Hz, 2H), 4.51 (dt, ²J_{H-F}=47.0 Hz, ³J_{H-H}= 4.5 Hz, 2H), 5.27 (bs, 2H), 7.56 (dd, ³J_{H-H}=8.5 Hz, ⁴J_{H-H}=1.0 Hz, 1H), 7.80-7.85 (m, 5H), 7.95 (ddd, ³J_{H-H}=7.5 Hz, ³J_{H-H}=7.0 Hz, ⁴J_{H-H}=1.0 Hz, 1H), 8.15-8.25 (m, 2H), 8.38 (ddd, ³J_{H-H}=8.0 Hz, ³J_{H-H}=7.0 Hz, ⁴J_{H-H}=1.0 Hz, 1H), 8.73 (d, ³J_{H-H}=8.0 Hz, 1H), 9.26 (d, ³J_{H-H}= 8.5 Hz, 1H), 9.32 (dd, ³J_{H-H}=8.0 Hz, ⁴J_{H-H}=1.5 Hz, 1H).
¹³C NMR ((CD₃)₂SO, 125 MHz): δ 29.7 (d, ³J_{C-F}=19.6 Hz), 51.3 (d, ³J_{C-F}=4.9 Hz), 81.0 (d, ¹J_{C-F}=162.3 Hz), 120.67, 120.7 (q, ¹J_{C-F}=320.25 Hz), 123.2, 124.5, 125.6, 126.0, 128.3, 129.3, 130.3, 130.4, 130.9, 131.4, 132.4, 132.6, 133.8, 134.5, 137.4, 164.4.
¹⁹F NMR ((CD₃)₂SO, 470 MHz): δ -77.7 (s, 3F), -220.9 (tt, ²J_{F-H}=47.1, ³J_{F-H}=27.3, 1F).

### Example 23. 3,6-Bis(dimethylamino)-10-(3-fluoropropyl)acridinium trifluoromethanesulfonate

3-Fluoropropropyl trifluoromethanesulfonate (0.167 g, 0.79 mmol) is added to a 50 ml round-bottom single-neck flask with a magnetic dipole. 12 ml DCM is added using a needle and syringe. The solution is cooled to a temperature of -56°C using a cooling bath (liquid nitrogen + ethyl acetate). A solution of Acridinium Orange (0.168 g, 0.63 mmol) in 12 ml DCM is added dropwise to the cooled solution over about 20 minutes, after which the contents of the flask are stirred for 72h. At the end of the reaction, the reaction mixture is concentrated to about 12 ml, and 12 ml of cold Et₂O is added. The mixture is left in the refrigerator for 30 min, after which the product is filtered under reduced pressure. 0.339 g of the title compound is obtained (89.7% yield).
¹H NMR ((CD₃)₂SO, 500 MHz): δ 2.20 (d, ³J_{H-F}=28.0, 2H), 3.19 (s, 12H), 4.50-4.80 (m, 4H), 6.48 (s, 2H), 7.12 (d, ³J_{H-H}=9 Hz, 2H), 7.75 (d, ³J_{H-H}=9 Hz, 2H), 8.57 (s, 1H).
¹³C NMR ((CD₃)₂SO, 125 MHz): δ 26.7 (d, ²J_{C-F}=19.5 Hz), 40.1, 43.2, 81.7 (d, ¹J_{C-F}=161.3 Hz), 92.0, 114.0, 116.2, 120.7 (q, ¹J_{C-F}=320 Hz), 132.8, 142.0, 142.7, 155.2.
¹⁹F NMR ((CD₃)₂SO, 470 MHz): δ -77.7 (s, 3F), -219.0 (tt, ²J_{F-H}=49.4 Hz, ³J_{H-H}=25.1 Hz, 1F).

### Example 24. 3,6-Bis(dimethylamino)-10-(6-trifluoromethylsulfonyloxyhexyl)-acridinium trifluoromethanesulfonate

1,16-Bis(trifluoromethanesulfonyloxy)hexane (0.434 g, 1.14 mmol) is added to a 50 ml round-bottom single-neck flask with a magnetic dipole. 6 ml DCM is added using a needle and syringe. The solution is cooled to a temperature of -56°C using a cooling bath (liquid nitrogen + ethyl acetate), and a solution of Acridinium Orange (0.285 g, 1.07 mmol) in 12 ml DCM is added dropwise to the cooled solution over about 20 minutes. The contents of the flask are stirred for 96h, and at the end of the reaction, 20 ml of cold Et₂O is added. The mixture is left in the refrigerator for 30 min, after which the precipitate formed is filtered off under reduced pressure to obtain 0.370 g of raw product with an estimated content of the title compound of approx. 33% (as determined based on signal integration in the ¹H NMR spectrum).
¹H NMR ((CD₃)₂SO, 500 MHz): δ 1.0-2.0 (m, 8H), 3.22 (s, 12H), 4.40 (bs, 2H), 4.53 (bs, 2H), 6.28 (s, 2H), 7.02 (d, J=7.5 Hz, 2H), 7.65 (d, J=8.0 Hz, 2H), 8.43 (s, 1H).
¹³C NMR ((CD₃)₂SO, 125 MHz): δ 22.7, 25.0, 29.1, 29.2, 32.4, 46.6, 69.6, 91.8, 113.9, 114.2, 116.4, 132.7, 132.8, 142.4, 155.2.
¹⁹F NMR ((CD₃)₂SO, 470 MHz): δ -77.8 (s, 3F).

### Example 25. The method of manufacturing ¹⁸F-radiolabelled compounds according to the invention using the method illustrated by the reaction sequence according to scheme I

¹⁸F-labelled derivatives are synthesised using the Modular Lab Standard (Eckert&Ziegler) synthesiser. The ¹⁸F radioisotope is produced using Siemens Eclipse cyclotron in the ¹⁸O(p,n)¹⁸F reaction. The resulting ¹⁸F⁻ is deposited on the anion-exchange column QMA, from which water enriched with H₂¹⁸O is recovered. The fluoride compound ¹⁸F⁻ is eluted from the QMA column to the reactor using 600 µl kryptofix solution (22 mg) with potassium carbonate (11.7 mg) using H₂O:ACN eluent (1:1). Solvents are distilled off under reduced pressure, and then residual water is removed through azeotropic distillation by adding 1 ml ACN twice. A precursor, i.e. a quaternary ammonium salt of a polycyclic aromatic amine having a leaving group (preferably a trifluoromethanesulfonate group) in the chain side substituent on the quaternary nitrogen atom (in the amount of 12 to 20 mg) in DCM, is added to the reactor. The reaction is carried out at a temperature of 90°C for 6.5 to 10 min. The reactor is cooled, and the solution is transferred to a collection vial. The reactor is then washed with a solution of acetate buffer, pH=5.2 supplemented with ethanol (12 to 28.5%), which is also transferred to the collection vial. The collected solution is applied on a semi-preparative HPLC column (250x10 mm, Luna, Phenomenex), using the acetate buffer, pH=5.2, mixed with ethanol as the mobile phase. For the phenanthridine derivative a mobile phase with 12.0% ethanol content is used, for the phenylphenanthridine derivative a mobile phase with 23.5% ethanol content is used, and for the acridine derivative a mobile phase with 28.5% ethanol content is used.

Based on the tests carried out with the use of standards, a product was obtained through collection of fractions from the semi-preparative column with a specified retention time. For the phenanthridine derivative, the product is collected directly into the final vial, while the acridine and phenylphenanthridine derivatives are transferred to a second reactor to remove excess ethanol through distillation at a temperature of 105-110°C for 8 to 10 min followed by transferring the product to the final vial. The labelling yield under these conditions is 1-5%.

The radioisotope-labelled compound according to the invention obtained in the form of acetate salt is analysed by high-performance liquid chromatography (HPLC) to confirm the identity (by comparison with the standard provided by a structural analogue of the radioisotope-labelled compound of the quaternary ammonium salt of a polycyclic aromatic amine according to the invention, wherein said analogue has a non-radioactive fluorine atom, i.e. ¹⁹F, replacing ¹⁸F at the corresponding position at R² substituent carbon atom) and to determine the level of chemical and/or radiochemical contaminants, if any. The identity of the ¹⁸F isotope is confirmed by determining half-life (110 min) and measuring the gamma radiation energy of the ¹⁸F isotope (main peak of 511 KeV). In addition, thin-layer chromatography (TLC) is used to determine radiochemical purity.

The method for manufacturing ¹⁸F-radiolabelled compounds according to the invention using the method as above also allows for obtaining the product in the form of a salt with a counterion other than the acetate anion. For this purpose, experiments were carried out using a semi-preparative HPLC column (250x10 mm, Luna, Phenomenex) used to obtain ¹⁸F-labelled compounds, on which column N-(2-fluoroethyl)-6-phenylphenanthridinium trifluoromethanesulfonate was loaded. Mixtures of acetonitrile and three different buffers of 0.1 M phosphoric acid (pH=2.4), ascorbic acid (pH=6.3) or citric acid (pH=6.3) concentration, respectively, in isocratic system, were used as eluents. Product samples obtained following the elution from the semi-preparative column were analysed by high resolution mass spectrometry to determine the structure of both the cation and anion of the eluted quaternary amine compound in the form of salt. The results of the analyses are summarised in table 1 below.

**Table 1**

| Buffer | Phosphate | Citrate | Ascorbate |
|---|---|---|---|
| pH | 2.4 | 6.3 | 6.3 |
| Phase composition (ACN% : buffer%) | 25:75 | 27:73 | 27:73 |
| Theoretical mass of the cation | 302.13395 | | |
| MS cation mass | 302.13381 | 302.13381 | 302.13377 |
| Anion | H₂PO₄⁻ | C₆H₇O₇⁻ | C₆H₇O₆⁻ |
| Theoretical mass of the anion | 96.96852 | 191.01863 | 175.02371 |
| MS anion mass | 96.96807 | 191.01870 | 175.02369 |

The results obtained clearly demonstrate that depending on the buffer added to the eluent used, the required counterion salt is obtained, and in these specific experiments a compound of quaternary ammonium salt of a polycyclic aromatic amine is obtained, wherein the ammonium cation is accompanied by a dihydrogen phosphate, citrate or ascorbate anion, respectively.

### Example 26. Manufacture of a pharmaceutical form of the ¹⁸F-radiolabelled compound according to invention

The synthesis, formulation and dispensing of the preparation are performed in hot chambers. The active ingredient, i.e. the radioisotope-labelled compound of a polycyclic quaternary aromatic amine according to the invention, is manufactured and purified in accordance with the procedure presented in the examples above, after which the appropriate pH of the active ingredient solution is determined using a buffer, e.g. citrate, acetate, phosphate buffer (optionally, if not necessary, no buffer is used). If required, a pharmacologically non-aqueous acceptable diluent/solvent is added to the solution (for example to improve solubility or stability, such as ethanol); optionally, the solution is not diluted. The resulting solution is added through a sterilisation filter, for example 0.22 µm in size, to a collection vial placed in a grade A purity isolator, where the final formulation and determination of the desired radioactive concentration is performed by way of dilution (if necessary) of the raw product with a physiological solution of sodium chloride or injection water. The resulting product in bulk is automatically distributed into the final containers (vials, or directly to syringes) through a 0.22 µm final sterilisation filter. Optionally, thermal final sterilisation is used where the thermal treatment does not adversely affect the stability of the product. The vials are placed in external protective shielding.

### Example 27. PET-CT scan performed using an animal model

Male rats weighing approx. 250 g are quarantined for a period of no less than 5 days. The animals are anaesthetised in an induction chamber using a 3.5-4% isoflurane (Baxter AErrane) atmoshpere. The anaesthetised animal is transferred under an anaesthesis-maintaining mask (1.5-2% isoflurane in the air). A catheter is mounted on the lateral caudal vein of the animal. A heparin solution (approx. 50 µl) is injected into the vein in order to check the patency of the catheter and prevent excessive blood clotting.

The animal is transferred to a measurement bed placed in a PET/SPECT/CT scanner (Albira Carestream) equipped with a sensor allowing for monitoring the number of breaths and a system providing anaesthesia during the measurement. The number of breaths during the measurement is regulated by the concentration of the isoflurane supplied in the air and maintained within the range of 50-70 breaths per minute. For the duration of the measurement, the eyes of the animal are protected with a protective preparation (Vidisic).

The animal is administered 100 to 200 µl of the ¹⁸F-labelled radiopharmaceutical of the compound according to the invention (a cardiotracer) and 100 to 200 µl saline solution (in order to transport as much cardiotracer as possible from the catheter's dead space).

When the tracer administration is over, the dynamic PET acquisition starts.

The PET acquisition takes 35 to 90 minutes, depending on the recommendations of the person ordering the scan. The acquisition consists of a sequence of scans with a duration of 30 to 500 s (shorter scans occur in the first phase of the acquisition due to the higher dynamics of changes in the tracer biodistribution).

After the PET acquisition is over, the test object is moved to the CT module, where the whole body scan is performed (45 kVp tube voltage, 400 mA current, 400 projections per rotation, 4 rotations).

After the acquisition is over, the animal is awakened from anaesthesia and placed in a cage for 48h in order to observe whether the tracer administration induced any adverse effects. It is then euthanised or may be used for another experiment, if planned within the next few days.

The acquisition results are reconstructed into 3D images using the Albira Suite Reconstructor software. An analysis is performed to obtain the specific uptake value (SUV) of the cardiotracer for selected organs and tissues: heart muscle, cardiac blood pool, lungs, liver, kidneys, bladder.

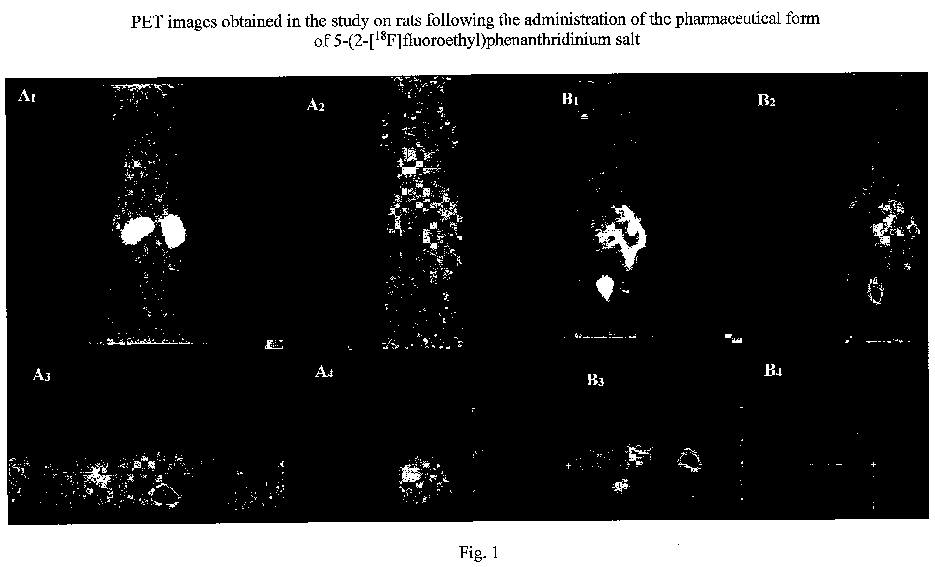

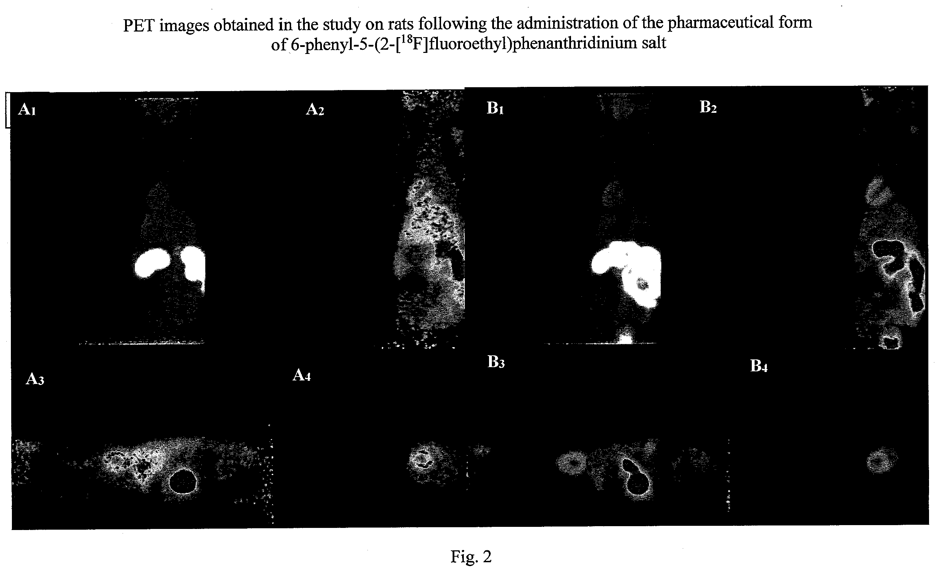

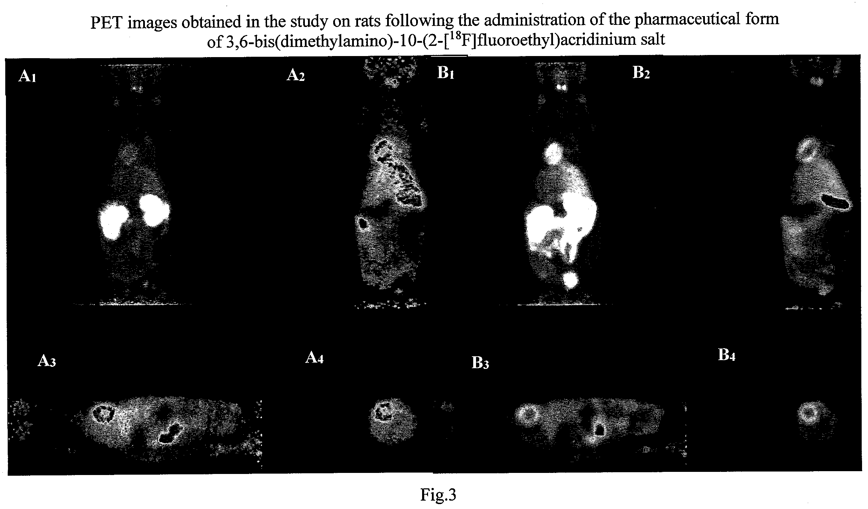

Fig. 1-3 illustrate representative PET images obtained in the above experimental animal studies: summed (1) and cross-sectional (2-4) in the frontal (1-2), sagittal (3) and transverse (4) plane of radioactivity distribution following the administration of compounds according to the subject invention to animals. More specifically, imaging of fig. 1 was obtained by administering the pharmaceutical form of 5-(2-[¹⁸F]fluoroethyl)phenanthridinium salt, imaging of fig. 2 was obtained by administering the pharmaceutical form of 6-phenyl-5-(2-[¹⁸F]fluoroethyl)phenanthridinium salt, and imaging of fig. 3 was obtained by administering the pharmaceutical form of 3,6-bis(dimethylamino)-10-(2-[¹⁸F]fluoroethyl)acridinium salt. The pharmaceutical forms provided in the study were solutions of acetate salts in saline aqueous solution (i.e. cations of the ammonium salts of a polycyclic aromatic amine with formula I according to the invention referred to above were accompanied by chloride and acetate anions). The images are presented as the sum of time frames: 2-5 min (A) and 20-35 min (B).

### Example 28. Description of the medical procedure in humans

Patients are placed on their back, with their hands behind their head, in the PET-CT scanner, and an intravenous catheter is placed in the lower part of the upper limb. Imaging begins with a resting scan in a dynamic data collection mode with the injection of a ¹⁸F-labelled radiopharmaceutical according to the invention. In the next scan, imaging is performed under stress following a pharmacological or physical stress. For dynamic imaging, the data acquisition start time is a few seconds before tracer administration. Perfusion images are acquired for 10 minutes directly following the intravenous tracer bolus administration. The minimum interval between the scan at rest and under stress is 50 minutes. Stress is induced in the patient through physical exercise or pharmacologically by administering regadenosone intravenous bolus (0.4 mg) regardless of body weight for 20-30 s. For regadenosone, the cannula is flushed with 5 ml saline directly after the administration, followed by the administration of a radioisotope-labelled compound according to the invention labelled with ¹⁸F (cardiotracer) 30 seconds after the saline. The PET scan is carried out as follows: 10 minutes of dynamic scan (12 × 10 seconds, 4 × 30 seconds, 1 × 6 minutes) in the area of the heart with an additional area above and below; 3D data acquisition, ECG gating (8 or 16 frames per cycle); array: 128 × 128.

PET imaging is performed at rest and under stress using PET-CT tomographs. With CT scans, attenuation adjustment is achieved 2 minutes before or after the test at rest and under stress. Low-dose CT is performed within 3 minutes before or after the acquisition of dynamic scans. The total CT scan time is 20 seconds. The rotation time of the X-ray tube is 0.5 second at 140 kV and 30 mA. Details of the myocardial perfusion imaging are summarised in table 2.

**Table 2. Rest and stress myocardial perfusion imaging**

| Characteristic | Imaging parameters |
|---|---|
| Stress conditions | Pharmacological agent regadenoson or physical strain |
| Tracer dose (3D) | (2-12 MBq/kg) usually 3-4 MBq/kg |
| Delay for static images | 1.5-3 minutes after the administration |
| Delay for dynamic images | Activating the device directly before injecting the tracer dose |
| PET/CT | CT scout |
| Imaging mode | ECG-gated imaging of perfusion and functional parameters of the myocardium |
| Mode: gated / dynamic | |
| Imaging duration | 12-15 minutes |
| Attenuation adjustment | Attenuation adjustment measurement before or after the acquisition |
| Reconstruction method | Iterative expectation maximisation method (e.g. OSEM) |
| Reconstruction filters | Sufficient to achieve the desired resolution/smoothing, matching stress and rest conditions |
| Reconstructed voxel size | 3.27 |

## Claims

1. A radioisotope-labelled compound of quaternary ammonium salt of a polycyclic aromatic amine with formula I, 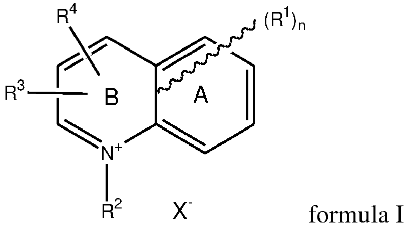 in which formula I
the wavy line indicates a single bond between the non-nodal carbon atom of a polycyclic aromatic system and the R¹substituent,
R¹ is a substituent independently selected from hydrogen, halogens, C₁₋₄alkoxy, a nitro group, an amino group optionally having 1 or 2 hydrogen atoms replaced with C₁₋₆alkyl or having 2 hydrogen atoms replaced with C₂₋₅alkylene to form a heterocyclic ring, a chain C₁₋₆carbon group optionally having a halogen, carboxy, formyl or C₁₋₄alkanesulfonic substituent, and a phenyl group optionally having 1-5 substituents independently selected from halogens, a chain C₁₋₆carbon, a halogenated chain C₁₋₆carbon substituent, C₁₋₄alkoxy, an amino group optionally having 1-2 atoms of hydrogen replaced with C₁₋₆alkyl,
R² is a chain aliphatic substituent having the -CH₂- fragment as the terminal member of the chain, to which chain a phenyl group is optionally attached optionally having 1-3 substituents selected from halogens and C₁₋₆alkyl, and, if the chain contains at least 2 carbon atoms, in which chain there is optionally a bivalent link between the chain carbon atoms, said link selected from the oxygen atom -O-, sulfur atom -S- and C₃₋₆cycloalkylene, wherein the R² substituent contains a total of 1-16 carbon atoms, and the hydrogen atom at one of the carbon atoms is replaced with an atom of ¹⁸F fluorine radioisotope,
R³ and R⁴ are combined to form a bivalent butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
or a hydrate or solvate thereof.

2. The radioisotope-labelled compound according to claim 1, wherein in formula I the wavy line indicates a single bond between the non-nodal carbon atom of a polycyclic aromatic system and the R¹ substituent,
R¹ is a substituent independently selected from hydrogen, halogens, C₁₋₄alkoxy, a nitro group, an amino group optionally having 1 or 2 hydrogen atoms replaced with C₁₋₄alkyl, a chain C₁₋₆carbon group optionally having a halogen or C₁₋₄alkanesulfonic substituent, and a phenyl group optionally having 1-3 substituents independently selected from halogens, a chain C₁₋₄carbon substituent, a halogenated chain C₁₋₄carbon substituent, C₁₋₄ alkoxy, an amino group optionally having 1-2 atoms of hydrogen replaced with C₁₋₄alkyl,
R² is a chain aliphatic substituent having the -CH₂- fragment as the terminal member of the chain, to which chain a phenyl group is optionally attached optionally having 1-3 substituents selected from halogens and C₁₋₆alkyl, and, if the chain contains at least 2 carbon atoms, in which chain there is optionally a bivalent link between the chain carbon atoms, said link selected from the oxygen atom -O- and sulfur atom -S-, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
or a hydrate or solvate thereof.

3. The radioisotope-labelled compound according to claim 2, wherein in formula I the wavy line indicates a single bond between the non-nodal carbon atom of a polycyclic aromatic system and the R¹ substituent,
R¹ is a substituent independently selected from hydrogen, halogens, C₁₋₄alkoxy, an amino group optionally having 1-2 hydrogen atoms replaced with C₁₋₄alkyl, a chain C₁₋₄carbon group optionally having a halogen substituent, and a phenyl group optionally having 1-3 substituents independently selected from halogens, a chain C₁₋₄carbon substituent, a halogenated chain C₁₋₄carbon substituent, C₁₋₄alkoxy,
R² is a chain aliphatic substituent having the -CH₂- fragment as the terminal member of the chain, to which chain a phenyl group is optionally attached optionally having 1-3 substituents selected from halogens and C₁₋₆alkyl, and, if the chain contains at least 2 carbon atoms, in which chain there is optionally a bivalent link between the chain carbon atoms, said link being the oxygen atom -O-, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
or a hydrate or solvate thereof.

4. The radioisotope-labelled compound according to claim 3, wherein in formula I the wavy line indicates a single bond between the non-nodal carbon atom of a polycyclic aromatic system and the R¹ substituent,
R¹ is a substituent independently selected from hydrogen, halogens, C₁₋₄alkoxy, an amino group optionally having 1-2 hydrogen atoms replaced with C₁₋₂alkyl, a chain C₁₋₄carbon group optionally having a halogen substituent, and a phenyl group optionally having 1-3 substituents independently selected from halogens, a chain C₁₋₄carbon substituent and a halogenated chain C₁₋₄carbon substituent,
R² is a chain aliphatic substituent having the -CH₂- fragment as the terminal member of the chain, to which chain a phenyl group is optionally attached optionally having 1-3 substituents selected from halogens and C₁₋₄alkyl, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
or a hydrate or solvate thereof.

5. The radioisotope-labelled compound according to claim 4, wherein in formula I the wavy line indicates a single bond between the non-nodal carbon atom of a polycyclic aromatic system and the R¹ substituent,
R¹ is a substituent independently selected from an atom of hydrogen, halogens, an amino group optionally having 1-2 hydrogen atoms replaced with C₁₋₂alkyl, a chain C₁₋₄carbon group and a phenyl group optionally having 1-3 substituents independently selected from halogens, a chain C₁₋₄carbon substituent, a halogenated chain C₁₋₄carbon substituent,
R² is a chain aliphatic substituent having the -CH₂- fragment as the terminal member of the chain, to which chain a phenyl group is optionally attached, optionally having a halogen substituent, wherein the R² substituent contains a total of 1-16 carbon atoms, and the hydrogen atom at one of the carbon atoms is replaced with an atom of ¹⁸F fluorine radioisotope,
R³ and R⁴ are combined to form a butadienyl-1,3 substituent whose terminal carbon atoms are linked to the adjacent non-nodal carbon atoms of the B ring to form an aromatic C ring fused with the A and B ring system, having R¹ substituents at non-nodal carbon atoms,
n is an integer of 9,
X⁻ is a pharmaceutically acceptable counterion, which is an anion of a mono-basic inorganic acid, a mononegative anion of a multi-basic inorganic acid, an anion of an alkanecarboxylic acid, an anion of an aliphatic sulfonic acid, an anion of an aromatic sulfonic acid, an anion of an acidic amino acid,
or a hydrate or solvate thereof.

6. The radioisotope-labelled compound according to any of claims 1 to 5 for use in a diagnostic method of positron emission tomography.

7. The radioisotope-labelled compound for use according to claim 6, wherein the diagnostic method is used for the examination of the cardiovascular system in a mammal.

8. The radioisotope-labelled compound for use according to claim 7, wherein the diagnostic method includes testing myocardial perfusion to quantify regional blood flow through the myocardium.

9. The radioisotope-labelled compound for use according to claim 7, wherein the diagnostic method includes testing myocardial perfusion to quantify the coronary reserve in coronary artery disease.

10. A pharmaceutical composition containing the radioisotope-labelled compound according to any of claims 1 to 5, and a pharmaceutically acceptable carrier or diluent.

11. The pharmaceutical composition according to claim 10, wherein it is in the form of a sterile solution.

12. The use of the radioisotope-labelled compound according to any of claims 1 to 5 for the manufacture of an agent for use in a diagnostic method of positron emission tomography.

13. The use according to claim 12, wherein the diagnostic method is used for the examination of the cardiovascular system in a mammal.

14. The use according to claim 13, wherein the diagnostic method is used for testing myocardial perfusion to quantify regional blood flow through the myocardium.

15. The use according to claim 14, wherein the diagnostic method is used for testing myocardial perfusion to quantify the coronary reserve in coronary artery disease.

## Patentansprüche

1. Radioisotopisch markierte Verbindung eines quaternären Ammoniumsalzes eines polyzyklischen aromatischen Amins mit der Formel I, 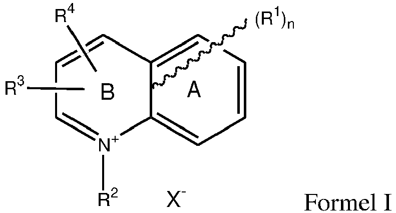 wobei die Formel I
die Wellenlinie zeigt eine einfache Verbindung zwischen dem nicht-nodalen Kohlenstoffatom eines polyzyklischen aromatischen Systems und dem R¹-Substituenten an,
R¹ ist ein Substituent, unabhängig ausgewählt aus Wasserstoff, Halogenen, C₁₋₄Alkoxy, einer Nitrogruppe, einer Aminogruppe, bei der gegebenenfalls 1 oder 2 Wasserstoffatome durch C₁₋₆Alkyl ersetzt sind oder bei denen 2 Wasserstoffatome durch C₂₋₅Alkylen ersetzt sind, um einen heterozyklischen Ring zu bilden, eine Kette der C₁₋₆Kohlenstoffgruppe, die gegebenenfalls ein Halogen, Carboxy, Formyl oder einen C₁₋₄Alkansulfon-Substituenten und eine Phenylgruppe, die gegebenenfalls 1-5 Substituenten aufweist, die unabhängig voneinander aus Halogenen, einer Kette des C₁₋₆Kohlenstoffs, einer halogenierten Kette des C₁₋₆Kohlenstoff-Substituenten, C₁₋₄Alkoxy, einer Aminogruppe, bei der gegebenenfalls 1-2 Wasserstoffatome durch C₁₋₆Alkyl ersetzt sind, gewählt wird,
R² ist ein kettenförmiger aliphatischer Substituent mit einem CH₂-Abschnitt als das Endglied der Kette, wobei an diese Kette gegebenenfalls eine Phenylgruppe angebunden ist, die gegebenenfalls 1-3 Substituenten aufweist, die aus Halogenen und C₁₋₆Alkyl ausgewählt sind, und, wenn die Kette mindestens 2 Kohlenstoffatome enthält, in welcher Kette gegebenenfalls eine zweiwertige Verbindung zwischen den Kettenkohlenstoffatomen vorhanden ist, wobei eine Verbindung mit dem Sauerstoffatom -O-, dem Schwefelatom -S- und C₃₋₆Cycloalkylen ausgewählt ist, wobei der Substituent R² insgesamt 1-16 Kohlenstoffatome enthält und wobei das Wasserstoffatom an einem der Kohlenstoffatome durch ein Atom des Radioisotops ¹⁸F-Fluor ersetzt ist,
R³ und R⁴ werden miteinander kombiniert, um einen bivalenten Butadienyl-1,3-Substituenten zu bilden, dessen endständige Kohlenstoffatome an die angrenzenden nicht-nodalen Kohlenstoffatome des B-Rings gebunden sind, um einen aromatischen C-Ring zu bilden, der mit dem A- und B-Ringsystem verschmolzen ist und die R¹-Substituenten an den nicht-nodalen Kohlenstoffatomen aufweist,
n ist eine ganze Zahl von 9,
X⁻ ist ein pharmazeutisch zulässiges Gegenion, das ein Anion einer einbasischen anorganischen Säure, ein mononegatives Anion einer mehrbasischen anorganischen Säure, ein Anion der Alkancarbonsäure, ein Anion der aliphatischen Sulfonsäure, ein Anion der aromatischen Sulfonsäure oder ein Anion der sauren Aminosäure ist,
oder ein Hydrat oder Solvat davon.

2. Die radioisotopisch markierte Verbindung nach Anspruch 1, wobei die Wellenlinie in der Formel I eine einfache Verbindung zwischen dem nicht-nodalen Kohlenstoffatom eines polyzyklischen aromatischen Systems und dem R¹-Substituenten anzeigt,
R¹ ist ein Substituent, unabhängig ausgewählt aus Wasserstoff, Halogenen, C₁₋₄-Alkoxy, einer Nitrogruppe, einer Aminogruppe, bei der gegebenenfalls 1 oder 2 Wasserstoffatome durch C₁₋₄-Alkyl ersetzt sind, eine Kette der C₁₋₆-Kohlenstoffgruppe, die gegebenenfalls ein Halogen oder einen C₁₋₄-Alkansulfon-Substituenten aufweist, und eine Phenylgruppe, die gegebenenfalls 1-3 Substituenten aufweist, die unabhängig voneinander aus Halogenen, einer Kette aus C₁₋₄-Kohlenstoff-Substituenten, einer halogenierten Kette C₁₋₄-Kohlenstoff-Substituenten, C₁₋₄-Alkoxy, einer Aminogruppe, bei der gegebenenfalls 1-2 Wasserstoffatome durch C₁₋₄alkyl ersetzt sind, besteht,
R² ist ein kettenförmiger aliphatischer Substituent mit dem CH₂-Abschnitt als Endglied der Kette, wobei an dieser Kette gegebenenfalls eine Phenylgruppe gebunden ist, die gegebenenfalls 1-3 Substituenten aufweist, die aus Halogenen und C₁₋₆-Alkyl ausgewählt sind, und wenn die Kette mindestens 2 Kohlenstoffatome enthält, in welcher Kette gegebenenfalls eine zweiwertige Verbindung zwischen den Kettenkohlenstoffatomen vorhanden ist, wobei die Verbindung mit dem Sauerstoffatom -O-, dem Schwefelatom -S- bestehen kann, wobei der Substituent R² insgesamt 1-16 Kohlenstoffatome enthält und das Wasserstoffatom an einem der Kohlenstoffatome durch ein Atom des Radioisotops ¹⁸F-Fluor ersetzt ist,
R³ und R⁴ werden miteinander kombiniert, um einen Butadienyl-1,3-Substituenten zu bilden, dessen endständige Kohlenstoffatome an die angrenzenden nicht-nodalen Kohlenstoffatome des B-Rings gebunden sind, um einen aromatischen C-Ring zu bilden, der mit dem A- und B-Ringsystem verschmolzen ist und die R¹-Substituenten an nicht-nodalen Kohlenstoffatomen aufweist,
n ist eine ganze Zahl von 9,
X⁻ ist ein pharmazeutisch annehmbares Gegenion, das ein Anion einer einbasischen anorganischen Säure, ein mononegatives Anion einer mehrbasischen anorganischen Säure, ein Anion einer Alkancarbonsäure, ein Anion einer aliphatischen Sulfonsäure, ein Anion einer aromatischen Sulfonsäure oder ein Anion einer sauren Aminosäure ist,
oder ein Hydrat oder Solvat davon.

3. Die radioisotopisch markierte Verbindung nach Anspruch 2, wobei die Wellenlinie in der Formel I eine einfache Verbindung zwischen dem nicht-nodalen Kohlenstoffatom eines polyzyklischen aromatischen Systems und dem R¹-Substituenten anzeigt,
R¹ ist ein Substituent, unabhängig ausgewählt aus Wasserstoff, Halogenen, C₁₋₄-Alkoxy, einer Aminogruppe, bei der gegebenenfalls 1-2 Wasserstoffatome durch C₁₋₄-Alkyl ersetzt sind, einer Kette der C₁₋₄-Kohlenstoffgruppe, die gegebenenfalls einen Halogen-Substituenten aufweist, und einer Phenylgruppe, die gegebenenfalls 1-3 Substituenten aufweist, die unabhängig voneinander aus Halogenen, einer Kette des C₁₋₄-Kohlenstoff-Substituenten, einer halogenierten Kette des C₁₋₄-Kohlenstoff-Substituenten, C₁₋₄-Alkoxy ausgewählt wurden,
R² ist ein kettenförmiger aliphatischer Substituent mit dem CH₂-Abschnitt als das Endglied der Kette, wobei an dieser Kette gegebenenfalls eine Phenylgruppe gebunden ist, die gegebenenfalls 1-3 Substituenten aufweist, die aus Halogenen und C₁₋₆-Alkyl ausgewählt sind, und, wenn die Kette mindestens 2 Kohlenstoffatome enthält, in welcher Kette gegebenenfalls eine zweiwertige Verbindung zwischen den Kettenkohlenstoffatomen vorhanden ist, wobei die Verbindung aus dem Sauerstoffatom -O- besteht, wobei der Substituent R² insgesamt 1-16 Kohlenstoffatome enthält und das Wasserstoffatom an einem der Kohlenstoffatome durch ein Atom des Radioisotops ¹⁸F-Fluor ersetzt ist,
R³ und R⁴ werden miteinander kombiniert, um einen Butadienyl-1,3-Substituenten zu bilden, dessen endständige Kohlenstoffatome an die angrenzenden nicht-nodalen Kohlenstoffatome des B-Rings gebunden sind, um einen aromatischen C-Ring zu bilden, der mit dem A- und B-Ringsystem verschmolzen ist und die R¹-Substituenten an den nicht-nodalen Kohlenstoffatomen aufweist,
n ist eine ganze Zahl von 9,
X⁻ ist ein pharmazeutisch annehmbares Gegenion, das ein Anion einer einbasischen anorganischen Säure, ein mononegatives Anion einer mehrbasischen anorganischen Säure, ein Anion der Alkancarbonsäure, ein Anion der aliphatischen Sulfonsäure, ein Anion der aromatischen Sulfonsäure oder ein Anion der sauren Aminosäure ist,
oder ein Hydrat oder Solvat davon.

4. Die radioisotopisch markierte Verbindung nach Anspruch 3, wobei die Wellenlinie in der Formel I eine einfache Verbindung zwischen dem nicht-nodalen Kohlenstoffatom eines polyzyklischen aromatischen Systems und dem R¹-Substituenten anzeigt,
R¹ ist ein Substituent, unabhängig ausgewählt aus Wasserstoff, Halogenen, C₁₋₄-Alkoxy, einer Aminogruppe, bei der gegebenenfalls 1-2 Wasserstoffatome durch C₁₋₂-Alkyl ersetzt sind, einer Kette der C₁₋₄-Kohlenstoffgruppe, die gegebenenfalls einen Halogensubstituenten aufweist, und einer Phenylgruppe, die gegebenenfalls 1-3 Substituenten aufweist, die aus Halogenen, einer Kette des C₁₋₄-Kohlenstoff-Substituenten und einer halogenierten Kette des C₁₋₄Kohlenstoff-Substituenten ausgewählt wurden,
R² ist ein kettenförmiger aliphatischer Substituent mit dem CH₂-Abschnitt als das Endglied der Kette, wobei an dieser Kette gegebenenfalls eine Phenylgruppe gebunden ist, die gegebenenfalls 1-3 Substituenten aufweist, die aus Halogenen und C₁₋₄-Alkyl ausgewählt sind, wobei der Substituent R² insgesamt 1-16 Kohlenstoffatome enthält und das Wasserstoffatom an einem der Kohlenstoffatome durch ein Atom des Radioisotops ¹⁸F-Fluor ersetzt ist,
R³ und R⁴ werden miteinander kombiniert, um einen Butadienyl-1,3-Substituenten zu bilden, dessen endständige Kohlenstoffatome an die angrenzenden nicht-nodalen Kohlenstoffatome des B-Rings gebunden sind, um einen aromatischen C-Ring zu bilden, der mit dem A- und B-Ringsystem verschmolzen ist und die R¹-Substituenten an nicht-nodalen Kohlenstoffatomen aufweist,
n ist eine ganze Zahl von 9,
X⁻ ist ein pharmazeutisch annehmbares Gegenion, das ein Anion einer einbasischen anorganischen Säure, ein mononegatives Anion einer mehrbasischen anorganischen Säure, ein Anion der Alkancarbonsäure, ein Anion der aliphatischen Sulfonsäure, ein Anion der aromatischen Sulfonsäure oder ein Anion der sauren Aminosäure ist,
oder ein Hydrat oder Solvat davon.

5. Die radioisotopisch markierte Verbindung nach Anspruch 4, wobei die Wellenlinie in der Formel I eine einfache Verbindung zwischen dem nicht-nodalen Kohlenstoffatom eines polyzyklischen aromatischen Systems und dem R¹-Substituenten anzeigt,
R¹ ist ein Substituent, unabhängig ausgewählt aus einem Wasserstoffatom, Halogenen, einer Aminogruppe, bei der gegebenenfalls 1-2 Wasserstoffatome durch C₁₋₂-Alkyl ersetzt sind, einer Kette der C₁₋₄Kohlenstoffgruppe und einer Phenylgruppe, die gegebenenfalls 1-3 Substituenten aufweist, die unabhängig voneinander aus Halogenen, einer Kette des C₁₋₄-Kohlenstoff-Substituenten, einer halogenierten Kette C₁₋₄-Kohlenstoff-Substituenten gewählt wurden,
R² ist ein kettenförmiger aliphatischer Substituent mit dem CH2-Abschnitt als das Endglied der Kette, wobei an dieser Kette gegebenenfalls eine Phenylgruppe angebunden ist, die gegebenenfalls einen Substituenten aufweist, das aus Halogenen ausgewählt sind, wobei der Substituent R² insgesamt 1-16 Kohlenstoffatome enthält und das Wasserstoffatom an einem der Kohlenstoffatome durch ein Atom des Radioisotops ¹⁸F-Fluor ersetzt ist,
R³ und R⁴ werden miteinander kombiniert, um einen Butadienyl-1,3-Substituenten zu bilden, dessen endständige Kohlenstoffatome an die angrenzenden nicht-nodalen Kohlenstoffatome des B-Rings gebunden sind, um einen aromatischen C-Ring zu bilden, der mit dem A- und B-Ringsystem verschmolzen ist und die R¹-Substituenten an nicht-nodalen Kohlenstoffatomen aufweist,
n ist eine ganze Zahl von 9,
X⁻ ist ein pharmazeutisch annehmbares Gegenion, das ein Anion einer einbasischen anorganischen Säure, ein mononegatives Anion einer mehrbasischen anorganischen Säure, ein Anion der Alkancarbonsäure, ein Anion der aliphatischen Sulfonsäure, ein Anion der aromatischen Sulfonsäure oder ein Anion der sauren Aminosäure ist,
oder ein Hydrat oder Solvat davon.

6. Die radioisotopisch markierte Verbindung nach einem der Ansprüche 1 bis 5 zur Verwendung in einem diagnostischen Verfahren der Positronen-Emissions-Tomographie.

7. Die radioisotopisch markierte Verbindung zur Verwendung nach Anspruch 6, wobei das diagnostische Verfahren für die Untersuchung des kardiovaskulären Systems von Säugetieren verwendet wird.

8. Die radioisotopisch markierte Verbindung zur Verwendung nach Anspruch 7, wobei das Diagnoseverfahren die Untersuchung der Myokardperfusion zur Quantifizierung des regionalen Blutflusses durch das Myokard umfasst.

9. Die radioisotopisch markierte Verbindung zur Verwendung nach Anspruch 7, wobei das diagnostische Verfahren die Untersuchung der Myokardperfusion zur Quantifizierung der kardiovaskulären Reserve bei der Koronarerkrankung eingesetzt wird.

10. Eine pharmazeutische Zubereitung, die radioisotopisch markierte Verbindung nach einem der Ansprüche 1 bis 5 und einen pharmazeutisch zulässigen Trägerstoff oder ein Verdünnungsmittel enthält.

11. Eine pharmazeutische Zubereitung nach Anspruch 10, wobei sie in Form einer sterilen Lösung vorliegt.

12. Die Verwendung der radioisotopisch markierten Verbindung nach einem der Ansprüche 1 bis 5 zur Herstellung eines Agenten für das diagnostische Verfahren der Positronen-Emissions-Tomographie.

13. Die Verwendung nach Anspruch 12, wobei das diagnostische Verfahren für die Untersuchung des kardiovaskulären Systems von Säugetieren verwendet wird.

14. Die Verwendung nach Anspruch 13, wobei das Diagnoseverfahren die Untersuchung der Myokardperfusion zur Quantifizierung des regionalen Blutflusses durch das Myokard verwendet wird.

15. Die Verwendung nach Anspruch 14, wobei das diagnostische Verfahren zur Untersuchung der Myokardperfusion zwecks Quantifizierung der kardiovaskulären Reserve bei der Koronarerkrankung eingesetzt wird.

## Revendications

1. Composé marqué par un radio-isotope d'un sel d'ammonium quaternaire d'une amine aromatique polycyclique de formule I, 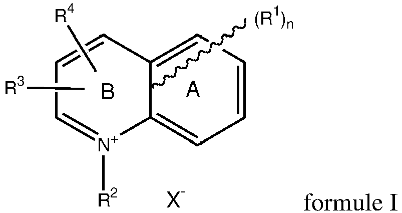 dans laquelle formule I
la ligne ondulée indique une liaison simple entre l'atome de carbone non nodal d'un système aromatique polycyclique et le substituant R¹,
R¹ est un substituant choisi indépendamment parmi l'hydrogène, les halogènes, les alcoxy en C₁₋₄, un groupe nitro, un groupe amino ayant éventuellement 1 ou 2 atomes d'hydrogène remplacés par un alkyle en C₁₋₆ ou ayant 2 atomes d'hydrogène remplacés par un alkylène en C₂₋₅ pour former un cycle hétérocyclique, un groupe à chaîne de carbone en C₁₋₆ ayant éventuellement un substituant halogène, carboxy, formyle ou alcanesulfonique en C₁₋₄, et un groupe phényle ayant éventuellement 1 à 5 substituants choisis indépendamment parmi les halogènes, une chaîne carbonée en C₁₋₆, un substituant halogéné de chaîne carbonée en C₁₋₆, un alcoxy en C₁₋₄, un groupe amino ayant éventuellement 1 ou 2 atomes d'hydrogène remplacés par un alkyle en C₁₋₆,
R² est un substituant aliphatique de la chaîne ayant le fragment -CH₂-comme élément terminal de la chaîne, chaîne à laquelle est éventuellement attaché un groupe phényle ayant éventuellement 1-3 substituants choisis parmi les halogènes et les alkyles en C₁₋₆, et, si la chaîne contient au moins 2 atomes de carbone, chaîne dans laquelle il y a éventuellement une liaison bivalente entre les atomes de carbone de la chaîne, ladite liaison étant choisie parmi l'atome d'oxygène -O-, l'atome de soufre -S- et le cycloalkylène en C₃₋₆, où le substituant R² contient un total de 1-16 atomes de carbone, et l'atome d'hydrogène à l'un des atomes de carbone est remplacé par un atome de radio-isotope de fluor ¹⁸F,
R³ et R⁴ sont combinés pour former un substituant bivalent butadiényl-1,3 dont les atomes de carbone terminaux sont liés aux atomes de carbone adjacents non nodaux du cycle B pour former un cycle aromatique C fusionné avec le système cyclique A et B, ayant des substituants R¹ sur les atomes de carbone non nodaux,
n est un nombre entier de 9,
X⁻ est un contre-ion pharmaceutiquement acceptable, qui est un anion d'un acide inorganique mono basique, un anion mononégatif d'un acide inorganique multi basique, un anion d'un acide alcanecarboxylique, un anion d'un acide sulfonique aliphatique, un anion d'un acide sulfonique aromatique, un anion d'un acide aminé acide,
ou un hydrate ou un solvate de celui-ci.

2. Composé marqué par un radio-isotope selon la revendication 1, dans lequel, dans la formule I, la ligne ondulée indique une liaison simple entre l'atome de carbone non nodal d'un système aromatique polycyclique et le substituant R¹,
R¹ est un substituant choisi indépendamment parmi l'hydrogène, les halogènes, les alcoxy en C₁₋₄, un groupe nitro, un groupe amino ayant éventuellement 1 ou 2 atomes d'hydrogène remplacés par un alkyle en C₁₋₄, un groupe à chaîne de carbone en C₁₋₆ ayant éventuellement un substituant halogène ou alcanesulfonique en C₁₋₄, et un groupe phényle ayant éventuellement 1-3 substituants choisis indépendamment parmi les halogènes, un substituant à chaîne de carbone en C₁₋₄, un substituant à chaîne de carbone en C₁₋₄ halogéné, un alcoxy en C₁₋₄, un groupe amino ayant éventuellement 1-2 atomes d'hydrogène remplacés par un alkyle en C₁₋₄,
R² est un substituant aliphatique de la chaîne ayant le fragment -CH₂-comme élément terminal de la chaîne, chaîne à laquelle est éventuellement attaché un groupe phényle ayant éventuellement 1-3 substituants choisis parmi les halogènes et les groupes alkyle en C₁₋₆, et, si la chaîne contient au moins 2 atomes de carbone, chaîne dans laquelle il y a éventuellement une liaison bivalente entre les atomes de carbone de la chaîne, ladite liaison étant choisie parmi l'atome d'oxygène -O- et l'atome de soufre -S-, où le substituant R² contient un total de 1-16 atomes de carbone, et l'atome d'hydrogène à l'un des atomes de carbone est remplacé par un atome de radio-isotope de fluor ¹⁸F,
R³ et R⁴ sont combinés pour former un substituant butadiényl-1,3 dont les atomes de carbone terminaux sont liés aux atomes de carbone non nodaux adjacents du cycle B pour former un cycle aromatique C fusionné avec le système de cycles A et B, ayant des substituants R¹ au niveau des atomes de carbone non nodaux,
n est un nombre entier de 9,
X⁻ est un contre-ion pharmaceutiquement acceptable, qui est un anion d'un acide inorganique monobasique, un anion mononégatif d'un acide inorganique multibasique, un anion d'un acide alcanecarboxylique, un anion d'un acide sulfonique aliphatique, un anion d'un acide sulfonique aromatique, un anion d'un acide aminé acide,
ou un hydrate ou un solvate de celui-ci.

3. Composé marqué par un radio-isotope selon la revendication 2, dans lequel, dans la formule I, la ligne ondulée indique une liaison simple entre l'atome de carbone non nodal d'un système aromatique polycyclique et le substituant R¹,
R¹ est un substituant choisi indépendamment parmi l'hydrogène, les halogènes, les alcoxy en C₁₋₄, un groupe amino ayant éventuellement 1 ou 2 atomes d'hydrogène remplacés par un alkyle en C₁₋₄, un groupe à chaîne en C₁₋₄ ayant éventuellement un substituant halogène, et un groupe phényle ayant éventuellement 1 à 3 substituants choisis indépendamment parmi les halogènes, un substituant à chaîne en C₁₋₄, un substituant à chaîne halogéné en C₁₋₄, un alcoxy en C₁₋₄,
R² est un substituant aliphatique en chaîne ayant le fragment -CH₂- en tant qu'élément terminal de la chaîne, chaîne à laquelle un groupe phényle est éventuellement attaché, ayant éventuellement 1 à 3 substituants choisis parmi les halogènes et les groupes alkyle en C₁₋₆, et, si la chaîne contient au moins 2 atomes de carbone, chaîne dans laquelle il y a éventuellement une liaison bivalente entre les atomes de carbone de la chaîne, ladite liaison étant l'atome d'oxygène -O-, dans laquelle le substituant R² contient un total de 1 à 16 atomes de carbone, et l'atome d'hydrogène à l'un des atomes de carbone est remplacé par un atome de radio-isotope de fluor ¹⁸F,
R³ et R⁴ sont combinés pour former un substituant butadiényl-1,3 dont les atomes de carbone terminaux sont liés aux atomes de carbone non nodaux adjacents du cycle B pour former un cycle aromatique C fusionné avec le système de cycles A et B, ayant des substituants R¹ sur les atomes de carbone non nodaux,
n est un entier de 9,
X⁻ est un contre-ion pharmaceutiquement acceptable, qui est un anion d'un acide inorganique mono-basique, un anion mononégatif d'un acide inorganique multi-basique, un anion d'un acide alcanecarboxylique, un anion d'un acide sulfonique aliphatique, un anion d'un acide sulfonique aromatique, un anion d'un acide aminé acide,
ou un hydrate ou un solvate de celui-ci.

4. Composé marqué par un radio-isotope selon la revendication 3, dans lequel, dans la formule I, la ligne ondulée indique une liaison simple entre l'atome de carbone non nodal d'un système aromatique polycyclique et le substituant R¹,
R¹ est un substituant choisi indépendamment parmi l'hydrogène, les halogènes, les alcoxy en C₁₋₄, un groupe amino ayant éventuellement 1-2 atomes d'hydrogène remplacés par un alkyle en C₁₋₂, un groupe carboné en C₁₋₄ à chaîne ayant éventuellement un substituant halogène, et un groupe phényle ayant éventuellement 1-3 substituants choisis indépendamment parmi les halogènes, un substituant carboné en C₁₋₄ à chaîne et un substituant carboné en C₁₋₄ à chaîne halogéné,
R² est un substituant aliphatique en chaîne ayant le fragment -CH₂- comme élément terminal de la chaîne, chaîne à laquelle un groupe phényle est facultativement attaché ayant facultativement 1-3 substituants choisis parmi les halogènes et un alkyle en C₁₋₄, où le substituant R² contient un total de 1-16 atomes de carbone, et l'atome d'hydrogène à l'un des atomes de carbone est remplacé par un atome de radio-isotope de fluor ¹⁸F,
R³ et R⁴ sont combinés pour former un substituant butadiényl-1,3 dont les atomes de carbone terminaux sont liés aux atomes de carbone non nodaux adjacents du cycle B pour former un cycle aromatique C fusionné avec le système de cycles A et B, ayant des substituants R¹ aux atomes de carbone non nodaux,
n est un nombre entier égal à 9,
n is an integer of 9,
X⁻ est un contre-ion pharmaceutiquement acceptable, qui est un anion d'un acide inorganique mono-basique, un anion mononégatif d'un acide inorganique multi-basique, un anion d'un acide alcanecarboxylique, un anion d'un acide sulfonique aliphatique, un anion d'un acide sulfonique aromatique, un anion d'un acide aminé acide,
ou un hydrate ou un solvate de celui-ci.

5. Composé marqué par un radio-isotope selon la revendication 4, dans lequel, dans la formule I, la ligne ondulée indique une liaison simple entre l'atome de carbone non nodal d'un système aromatique polycyclique et le substituant R¹,
R¹ est un substituant choisi indépendamment parmi un atome d'hydrogène, des halogènes, un groupe amino ayant éventuellement 1 ou 2 atomes d'hydrogène remplacés par un alkyle en C₁₋₂, un groupe à chaîne en C₁₋₄ et un groupe phényle ayant éventuellement 1 à 3 substituants choisis indépendamment parmi des halogènes, un substituant à chaîne en C₁₋₄, un substituant à chaîne halogéné en C₁₋₄,
R² est un substituant aliphatique en chaîne ayant le fragment -CH₂- comme élément terminal de la chaîne, chaîne à laquelle est éventuellement attaché un groupe phényle, ayant éventuellement un substituant halogène, dans lequel le substituant R² contient un total de 1-16 atomes de carbone, et l'atome d'hydrogène à l'un des atomes de carbone est remplacé par un atome de radio-isotope de fluor ¹⁸F,
R³ et R⁴ sont combinés pour former un substituant butadiényl-1,3 dont les atomes de carbone terminaux sont liés aux atomes de carbone non nodaux adjacents du cycle B pour former un cycle aromatique C fusionné avec le système de cycles A et B, ayant des substituants R¹ sur les atomes de carbone non nodaux,
n est un nombre entier égal à 9,
X⁻ est un contre-ion pharmaceutiquement acceptable, qui est un anion d'un acide inorganique mono-basique, un anion mononégatif d'un acide inorganique multi-basique, un anion d'un acide alcanecarboxylique, un anion d'un acide sulfonique aliphatique, un anion d'un acide sulfonique aromatique, un anion d'un acide aminé acide,
ou un hydrate ou un solvate de celui-ci.

6. Composé marqué par un radio-isotope selon l'une quelconque des revendications 1 à 5, destiné à être utilisé dans une méthode de diagnostic par tomographie par émission de positrons.

7. Composé marqué par un radio-isotope à utiliser selon la revendication 6, dans lequel la méthode de diagnostic est utilisée pour l'examen du système cardiovasculaire chez un mammifère.

8. Composé marqué par un radio-isotope à utiliser selon la revendication 7, dans lequel la méthode de diagnostic comprend le test de perfusion myocardique pour quantifier le flux sanguin régional à travers le myocarde.

9. Composé marqué par un radio-isotope à utiliser selon la revendication 7, dans lequel la méthode de diagnostic comprend le test de perfusion myocardique pour quantifier la réserve coronaire dans la maladie coronarienne.

10. Composition pharmaceutique contenant le composé marqué par un radio-isotope selon l'une quelconque des revendications 1 à 5, et un support ou un diluant pharmaceutiquement acceptable.

11. Composition pharmaceutique selon la revendication 10, dans laquelle elle se présente sous la forme d'une solution stérile.

12. Utilisation du composé marqué par un radio-isotope selon l'une quelconque des revendications 1 à 5 pour la fabrication d'un agent destiné à être utilisé dans une méthode de diagnostic de tomographie par émission de positrons.

13. Utilisation selon la revendication 12, dans laquelle la méthode de diagnostic est utilisée pour l'examen du système cardiovasculaire chez un mammifère.

14. Utilisation selon la revendication 13, dans laquelle la méthode de diagnostic est utilisée pour tester la perfusion myocardique afin de quantifier le flux sanguin régional à travers le myocarde.

15. Utilisation selon la revendication 14, dans laquelle la méthode de diagnostic est utilisée pour tester la perfusion myocardique afin de quantifier la réserve coronaire en cas de maladie coronarienne.
